# EUROPÄISCHE PATENTSCHRIFT

(11) **EP 2 958 971 B1**
(45) Veröffentlichungstag und Bekanntmachung des Hinweises auf die Patenterteilung: **11.07.2018**
(21) Anmeldenummer: 14705544.6
(22) Anmeldetag: 21.02.2014
(51) Int. Cl.: C09J 171/02, A61K 6/00

(54) **MEHRKOMPONENTENKLEBSTOFF ZUR HERSTELLUNG EINES ADHÄSIVHYDROGELS**
MULTI COMPONENT ADHESIVE FOR THE PREPARATION OF ADHESIVE HYDROGELS
ADHÉSIF À MULTIPLE COMPOSANTS POUR LA PREPARATION DE HYDROGELS

(30) Priorität: 21.02.2013 DE 102013202874
(43) Veröffentlichungstag der Anmeldung: 30.12.2015
(73) Patentinhaber: Fraunhofer-Gesellschaft zur Förderung der angewandten Forschung e.V., 80686 München (DE); Technische Universität Darmstadt, 64289 Darmstadt (DE); Johann Wolfgang Goethe-Universität, 60325 Frankfurt am Main (DE)
(72) Erfinder: RISCHKA, Klaus, 21255 Tostedt (DE); SADER, Robert, 60528 Frankfurt (DE); BARGEL, Hendrik, 95444 Bayreuth (DE); KOZIELEC, Maria, 22457 Hamburg (DE)
(74) Vertreter: Eisenführ Speiser
(86) Internationale Anmeldenummer: PCT/EP2014/053469
(87) Internationale Veröffentlichungsnummer: WO 2014/128272

(56) Entgegenhaltungen:
- US-A1- 2004 096 507
- US-A1- 2010 137 902
- EDWARD A. PHELPS ET AL: "Maleimide Cross-Linked Bioactive PEG Hydrogel Exhibits Improved Reaction Kinetics and Cross-Linking for Cell Encapsulation and In Situ Delivery", ADVANCED MATERIALS, Bd. 24, Nr. 1, 16. Dezember 2011 (2011-12-16), Seiten 64-70, XP055110903, ISSN: 0935-9648, DOI: 10.1002/adma.201103574
- DATABASE BIOSIS [Online] BIOSCIENCES INFORMATION SERVICE, PHILADELPHIA, PA, US; 2010, RISCHKA KLAUS ET AL: "Bio-inspired Polyphenolic Adhesives for Medical and Technical Applications", XP009177232, Database accession no. PREV201100321970 & RISCHKA KLAUS ET AL: "Bio-inspired Polyphenolic Adhesives for Medical and Technical Applications", BIOLOGICAL ADHESIVE SYSTEMS: FROM NATURE TO TECHNICAL AND MEDICAL APPLICATION SPRINGER-VERLAG BERLIN, HEIDELBERGER PLATZ 3, D-14197 BERLIN, GERMANY, 2010, Seiten 201-211,

### SEQUENCE LISTING

<110> Fraunhofer-Gesellschaft zur Förderung der angewandten Forschung e.V. Technische Universität Darmstadt
   Johann Wolfgang Goethe-Universität
<120> Mehrkomponentenklebstoff zur Herstellung eines Adhäsivhydrogels
<130> FA 1354-01DE
<140> not yet knwon
   <141> 2014-02-21
<150> DE10 2013 202 874.1
   <151> 2013-02-21
<160> 16
<170> BiSSAP 1.0
<210> 1
   <211> 4
   <212> PRT
   <213> artificial sequences
<220>
   <221> SOURCE
   <222> 1..4
   <223> /mol_type="protein"
   /note="Alanin-Lysin-Prolin-Serin"
   /organism="artificial sequences"
<400> 1
   Ala Lys Pro Ser
   1
<210> 2
   <211> 4
   <212> PRT
   <213> artificial sequences
<220>
   <221> SOURCE
   <222> 1..4
   <223> /mol_type="protein"
   /note="Lysin-Prolin-Serin-Tyrosin"
   /organism="artificial sequences"
<400> 2
   Lys Pro Ser Tyr
   1
<210> 3
   <211> 4
   <212> PRT
   <213> artificial sequences
<220>
   <221> SOURCE
   <222> 1..4
   <223> /mol_type="protein"
   /note="Prolin-Serin-Tyrosin-Prolin"
   /organism="artificial sequences"
<400> 3
   Pro Ser Tyr Pro
   1
<210> 4
   <211> 4
   <212> PRT
   <213> artificial sequences
<220>
   <221> SOURCE
   <222> 1..4
   <223> /mol_type="protein"
   /note="Serin-Tyrosin-Prolin-Prolin"
   /organism="artificial sequences"
<400> 4
   Ser Tyr Pro Pro
   1
<210> 5
   <211> 4
   <212> PRT
   <213> artificial sequences
<220>
   <221> SOURCE
   <222> 1..4
   <223> /mol_type="protein"
   /note="Tyrosin-Prolin-Prolin-Threonin"
   /organism="artificial sequences"
<400> 5
   Tyr Pro Pro Thr
   1
<210> 6
   <211> 5
   <212> PRT
   <213> artificial sequences
<220>
   <221> SOURCE
   <222> 1..5
   <223> /mol_type="protein"
   /note="Alanin-Lysin-Prolin-Serin-Tyrosin"
   /organism="artificial sequences"
<400> 6
   Ala Lys Pro Ser Tyr
   1               5
<210> 7
   <211> 5
   <212> PRT
   <213> artificial sequences
<220>
   <221> SOURCE
   <222> 1..5
   <223> /mol_type="protein"
   /note="Lysin-Prolin-Serin-Tyrosin-Prolin" /organism="artificial sequences"
<400> 7
   Lys Pro Ser Tyr Pro
   1               5
<210> 8
   <211> 5
   <212> PRT
   <213> artificial sequences
<220>
   <221> SOURCE
   <222> 1..5
   <223> /mol_type="protein"
   /note="Prolin-Serin-Tyrosin-Prolin-Prolin"
   /organism="artificial sequences"
<400> 8
   Pro Ser Tyr Pro Pro
   1               5
<210> 9
   <211> 5
   <212> PRT
   <213> artificial sequences
<220>
   <221> SOURCE
   <222> 1..5
   <223> /mol_type="protein"
   /note="Serin-Tyrosin-Prolin-Prolin-Threonin"
   /organism="artificial sequences"
<400> 9
   Ser Tyr Pro Pro Thr
   1               5
<210> 10
   <211> 6
   <212> PRT
   <213> artificial sequences
<220>
   <221> SOURCE
   <222> 1..6
   <223> /mol_type="protein"
   /note="Alanin-Lysin-Prolin-Serin-Tyrosin-Prolin"
   /organism="artificial sequences"
<400> 10
   Ala Lys Pro Ser Tyr Pro
   1               5
<210> 11
   <211> 6
   <212> PRT
   <213> artificial sequences
<220>
   <221> SOURCE
   <222> 1..6
   <223> /mol_type="protein"
   /note="Lysin-Prolin-Serin-Tyrosin-Prolin-Prolin"
   /organism="artificial sequences"
<400> 11
   Lys Pro Ser Tyr Pro Pro
   1               5
<210> 12
   <211> 6
   <212> PRT
   <213> artificial sequences
<220>
   <221> SOURCE
   <222> 1..6
   <223> /mol_type="protein"
   /note="Prolin-Serin-Tyrosin-Prolin-Prolin-Threonin"
   /organism="artificial sequences"
<400> 12
   Pro Ser Tyr Pro Pro Thr
   1               5
<210> 13
   <211> 7
   <212> PRT
   <213> artificial sequences
<220>
   <221> SOURCE
   <222> 1..7
   <223> /mol_type="protein"
   /note="Alanin-Lysin-Prolin-Serin-Tyrosin-Prolin-Prolin"
   /organism="artificial sequences"
<400> 13
   Ala Lys Pro Ser Tyr Pro Pro
   1               5
<210> 14
   <211> 7
   <212> PRT
   <213> artificial sequences
<220>
   <221> SOURCE
   <222> 1..7
   <223> /mol_type="protein"
   /note="Lysin-Prolin-Serin-Tyrosin-Prolin-Prolin-Threonin"
   /organism="artificial sequences"
<400> 14
   Lys Pro Ser Tyr Pro Pro Thr
   1               5
<210> 15
   <211> 8
   <212> PRT
   <213> artificial sequences
<220>
   <221> SOURCE
   <222> 1..8
   <223> /mol_type="protein"
   /note="Alanin-Lysin-Prolin-Serin-Tyrosin-Prolin-Prolin-Threonin"
   /organism="artificial sequences"
<400> 15
   Ala Lys Pro Ser Tyr Pro Pro Thr
   1               5
<210> 16
   <211> 11
   <212> PRT
   <213> artificial sequences
<220>
   <221> SOURCE
   <222> 1..11
   <223> /mol_type="protein"
   /note="Sequenz entspricht der Verbindung der Formel (IV); Cystein-Alanin-Lysin-Prolin-Serin-Tyrosin-Prolin-Prolin-Threonin-LDOPA-Ly sin"
   /organism="artificial sequences"
<220>
   <221> MOD_RES
   <222> 10
   <223> Xaa in Position 10 entspricht L-DOPA (L-3,4-Dihydroxyphenylalanin)
<400> 16
   Cys Ala Lys Pro Ser Tyr Pro Pro Thr Xaa Lys
   1               5                   10

## Beschreibung

Die vorliegende Erfindung betrifft primär einen Mehrkomponentenklebstoff zur Herstellung eines Adhäsivhydrogels, umfassend die nachfolgend definierte Verbindung der Formel (I) wie im Anspruch 1 definiert sowie eine, zwei, drei, vier oder mehr weitere Bestandteile. Die Erfindung betrifft zudem die Verwendung des besagten Mehrkomponentenklebstoffes und entsprechende Verfahren zum Zusammenkleben von zwei Oberflächen. Ferner betrifft die vorliegende Erfindung ein Verfahren zur Herstellung eines entsprechenden Adhäsivhydrogels. Die Erfindung betrifft auch ein Kit umfassend einen entsprechenden Mehrkomponentenklebstoff sowie einen oder mehrere weitere Bestandteile. Ebenfalls betrifft die Erfindung Artikel, die einen erfindungsgemäßen Mehrkomponentenklebstoff bzw. ein daraus hergestelltes Adhäsivhydrogel umfassen.

Weitere Aspekte der vorliegenden Erfindung ergeben sich aus der nachfolgenden Beschreibung sowie insbesondere aus den beigefügten Patentansprüchen.

In der modernen Chirurgie besteht ein steter Bedarf an neuen biokompatiblen Klebstoffen zum Verkleben von Gewebe. Ein wichtiges Anwendungsgebiet für solche biokompatiblen Klebstoffe liegt im Bereich der dentalen Implantologie. Beim Einsatz von Implantaten besteht trotz sorgfältiger Vorgehensweise seitens des (Zahn-)Arztes stets die Gefahr, dass Bakterien in die Grenzfläche zwischen Implantat und das das Implantat umgebende Gewebe (z. B. periimplantäre Schleimhaut) eindringen und zu Infektionen führen. Dabei sind Infektionen der periimplantären Schleimhaut (Mukosa) ohne Beteiligung des Knochens reversibel und werden als Mukositis bezeichnet. Bei fortschreitender Entzündung mit progressivem, irreversiblem Knochenverlust spricht man von einer Periimplantitis. Aufgrund des Knochenverlustes bei der Periimplantitis, führt diese zu einer Implantatlockerung und kann letztendlich auch zum Implantatverlust führen. Sowohl die Mukositis wie auch die Periimplantitis können derzeit nur durch regelmäßige Kontrollen und entsprechende therapeutische Maßnahmen nach der Diagnose erster Anzeichen für eine Mukositis vermieden werden. Dies ist mit einem hohen Behandlungsaufwand für den behandelnden (Zahn-)Arzt und Patienten und mit hohen Kosten verbunden.

Eine Stabilisierung bzw. Abdichtung der Schleimhaut und Gewebeschichten am Implantatkörper, die durch die Verwendung von biokompatiblen Klebstoffen erreicht wird, führt nicht nur zu einer stark verringerten Gefahr der Mukositis bzw. Periimplantitis, sondern verringert auch die Einheilzeit der Implantate an dem das Implantat umgebenden Gewebe (z. B. periimplantäres Stützgewebe). Eine wichtige Voraussetzung für eine erfolgreiche Implantation ist, dass das Implantat nach dem Einbringen in den Knochen eine ausreichende Primärstabilität besitzt. Das heißt, dass es direkt nach dem Einbringen nicht locker sitzen darf, sondern absolut, d. h. im maximal möglichen Ausmaß, fest im Knochen verankert ist. Durch ein entsprechendes Verkleben wird diese Primärstabilität unterstützt. Einwirkende Kaukräfte auf die Implantat-Knochenstruktur, die sich nach dem Einsetzen des Implantats im Aufbau und Umbau befindet, wirken aufgrund der Stabilisierung nicht in entscheidendem Maße auf neu entstandenes Gewebe, sodass dieses nicht wieder zerstört wird. Durch eine Stabilisierung bzw. Abdichtung der Implantate an dem das Implantat umgebenden Gewebe hat die orale Rekonstruktion auf lange Sicht eine sehr gute Ausgangssituation für eine vollkommene Rehabilitation. In der Folge kann der bislang übliche Behandlungsaufwand eines periimplantären Stützgewebeeinbruchs und die damit verbundenen Kosten reduziert bzw. völlig vermieden werden. Verglichen mit den Implantationslebenszeiten von bisherigen Behandlungsverfahren ohne biokompatiblen Klebstoffen, führt die Verwendung von biokompatiblen Klebstoffen auch zu deutlich längeren Implantationsüberlebenszeiten.

Neben der Anwendung von biokompatiblen Klebstoffen in der Implantologie sind auch weitere chirurgische, therapeutische und sonstige Anwendungen möglich. So lassen sich beispielsweise Schnittwunden durch das Auftragen entsprechender Klebstoffe verkleben. Auch das Kleben von Knochen-, Knorpel- und/oder Weichgewebe ist möglich.

Somit kann z. B. eine Fixierung von Knochenfrakturen erreicht werden, bei der die anschließenden Heilungsvorgänge deutlich schneller ablaufen können. Dabei nimmt die Eigenbelastbarkeit des Knochengewebes allmählich zu und der biokompatible Klebstoff wird allmählich abgebaut, bis nur noch reines Zellgewebe, bzw. Knochenmaterial vorhanden ist.

In der Literatur sind zahlreiche Klebstoffe und Abdichtmaterialien offenbart, die zur medizinischen Verwendung geeignet sind.

EP 1 609 431 B1 offenbart eine zellokklusive Membran, die durch Reaktion von zumindest zwei Vorläufermolekülen erhalten wird. Bei der beschriebenen zellokklusiven Membran handelt es sich nicht um einen Klebstoff, jedoch kann die Membran auch bei der Knochenregeneration oder in der Geweberegeneration verwendet werden.

In Bubaker et al., Biomaterials 31 (2010) 420 wird ein medizinischer Klebstoff offenbart. Der beschriebene Klebstoff kann durch Oxidation mit Natriumperiodat in ein Hydrogel überführt werden. Das Verkleben von Fettgewebe und Lebergewebe bei Mäusen wird ebenfalls beschrieben.

Bruce Lee et al., Macromolecules 39 (2006) 1740 offenbart einen Klebstoff, der durch Lichthärtung in ein Gel überführt werden kann. Der Klebstoff und das daraus resultierende Gel weisen einen hohen Gehalt an 3,4-Dihydroxyphenylanalin (DOPA) auf. Als mögliches Anwendungsbeispiel der offenbarten Klebstoffe bzw. der daraus gebildeten Gele wird die Verwendung als Bioklebstoff beschrieben.

WO 2012/009664 A2 offenbart medizinische Klebstoffe und Antifouling-Beschichtungen auf Basis von Proteinen der natürlichen marinen Muschelklebstoffe.

Aufgabe der vorliegenden Erfindung war es, einen Klebstoff zur Verfügung zu stellen, der nach dem Aushärten (Abbinden) zumindest eine, vorzugsweise jedoch mehrere, der nachfolgenden Eigenschaften aufweist:
- gute Biokompatibilität
- keine bzw. geringe Toxizität
- biologische Abbaubarkeit
- Fähigkeit zur Aufnahme und/oder zum Einbetten von Wirkstoffen sowie gegebenenfalls Fähigkeit zur zeitversetzten (Wieder-)Freigabe von aufgenommenen bzw. eingebetteten Wirkstoffen
- keine Immunreaktion im tierischen und/oder menschlichen Körper
- gute Adhäsion an biologischem Gewebe allogenen, heterogenen oder synthetischen Ursprungs und/oder an medizinischen Implantaten
- gute Barrierewirkung gegen Bakterien.

Gelöst wird diese Aufgabe durch einen Mehrkomponentenklebstoff zur Herstellung eines Adhäsivhydrogels, umfassend
a) eine Verbindung der Formel (I) 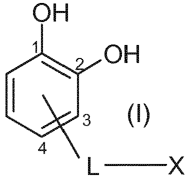 wobei
   X ausgewählt ist aus der Gruppe bestehend aus SH, NH₂ und alpha,beta-ungesättigten Carbonylgruppen und
   L ein Linker ist, der die 1,2-Dihydroxyphenyl-Einheit mit X verknüpft, wobei der Linker L in 3- oder 4-Position mit der 1,2-Dihydroxyphenyl-Einheit verbunden ist, und die Anzahl der Kettenglieder des Linkers L im Bereich von 5 bis 65 Kettengliedern liegt und der Linker L in der Verbindung der Formel (I) ein, zwei oder mehr Struktureinheiten enthält, ausgewählt aus der Gruppe von Aminosäuresequenzen bestehend aus Alanin-Lysin, Lysin-Prolin, Prolin-Serin, Serin-Tyrosin, Tyrosin-Prolin, Prolin-Prolin, Prolin-Threonin, Alanin-Lysin-Prolin, Lysin-Prolin-Serin, Prolin-Serin-Tyrosin, Serin-Tyrosin-Prolin, Tyrosin-Prolin-Prolin, Prolin-Prolin-Threonin, Alanin-Lysin-Prolin-Serin (SEQ ID NO: 1), Lysin-Prolin-Serin-Tyrosin (SEQ ID NO: 2), Prolin-Serin-Tyrosin-Prolin (SEQ ID NO: 3), Serin-Tyrosin-Prolin-Prolin (SEQ ID NO: 4), Tyrosin-Prolin-Prolin-Threonin (SEQ ID NO: 5), Alanin-Lysin-Prolin-Serin-Tyrosin (SEQ ID NO: 6), Lysin-Prolin-Serin-Tyrosin-Prolin (SEQ ID NO: 7), Prolin-Serin-Tyrosin-Prolin-Prolin (SEQ ID NO: 8), Serin-Tyrosin-Prolin-Prolin-Threonin (SEQ ID NO: 9), Alanin-Lysin-Prolin-Serin-Tyrosin-Prolin (SEQ ID NO: 10), Lysin-Prolin-Serin-Tyrosin-Prolin-Prolin (SEQ ID NO: 11), Prolin-Serin-Tyrosin-Prolin-Prolin-Threonin (SEQ ID NO: 12), Alanin-Lysin-Prolin-Serin-Tyrosin-Prolin-Prolin (SEQ ID NO: 13), Lysin-Prolin-Serin-Tyrosin-Prolin-Prolin-Threonin (SEQ ID NO: 14) und Alanin-Lysin-Prolin-Serin-Tyrosin-Prolin-Prolin-Threonin (SEQ ID NO: 15)
   und
b) eine, zwei, drei, vier oder mehr Verbindungen, jeweils enthaltend eine, zwei, drei, vier, fünf, sechs, sieben, acht oder mehr Struktureinheiten (IIa) 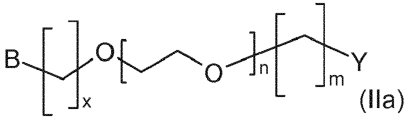 wobei in jeder der Struktureinheiten (IIa) unabhängig von der Bedeutung in anderen Struktureinheiten (IIa)
   B ein Verknüpfungspunkt ist,
   n eine ganze Zahl größer oder gleich 1 ist, x 0, 1, 2, 3, 4, 5, 6, 7 oder 8 ist und
   m 0, 1, 2, 3, 4, 5, 6, 7, oder 8 ist.Y ausgewählt ist aus der Gruppe bestehend aus SH, NH₂ und alpha,beta-ungesättigten Carbonylgruppen und umsetzbar ist mit der Gruppe X unter Ausbildung zumindest einer kovalenten Bindung,
   wobei
   (i) eine, zwei, drei, vier oder mehr der Verbindungen, die jeweils eine, zwei, drei, vier, fünf, sechs, sieben, acht oder mehrere Struktureinheiten (IIa) enthalten, zusätzlich jeweils einen Rest Z umfassen, der unabhängig von jedem anderen Rest Z einer anderen Verbindung ausgewählt ist aus der Gruppe bestehend aus SH, NH₂ und alpha,beta-ungesättigten Carbonylgruppen und umsetzbar ist mit einer Gruppe Y unter Ausbildung zumindest einer kovalenten Bindung,
      wobei in oder als Bestandteil (b) umfasst sind eine, zwei, drei, vier, fünf, sechs, sieben, acht oder mehr Verbindungen ausgewählt aus Verbindungen der Formel (V) 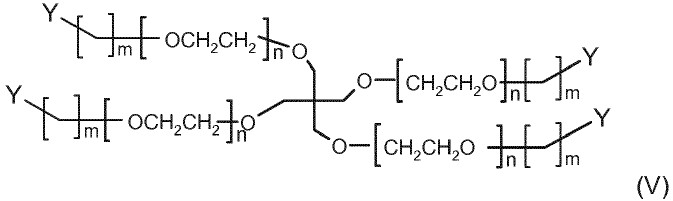 wobei
      n in jeder der vier Seitenketten unabhängig von der Bedeutung von n in den jeweils anderen Seitenketten eine ganze Zahl größer oder gleich 1 ist und
      m in jeder der vier Seitenketten unabhängig von der Bedeutung von m in den jeweils anderen Seitenketten 0, 1, 2, 3, 4, 5, 6, 7, oder 8 ist
         und
      Y in jeder der vier Seitenketten unabhängig von der Bedeutung von Y in den jeweils anderen Seitenketten ausgewählt ist aus der Gruppe bestehend aus SH, NH₂ und alpha,beta-ungesättigten Carbonylgruppen und umsetzbar ist mit der Gruppe X unter Ausbildung zumindest einer kovalenten Bindung.
      und/oder
   (ii) der Mehrkomponentenklebstoff zusätzlich eine, zwei, drei, vier oder mehr Verbindungen umfasst, die unabhängig voneinander jeweils zwei, drei, vier, fünf, sechs, sieben, acht oder mehr Struktureinheiten (IIIa) 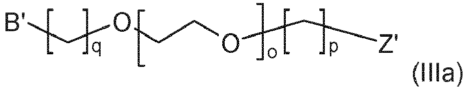 umfassen, wobei in jeder der Struktureinheiten (IIIa) unabhängig von der Bedeutung in anderen Struktureinheiten (IIIa)
      B' ein Verknüpfungspunkt ist,
      o eine ganze Zahl größer oder gleich 1 ist,
      q 0, 1, 2, 3, 4, 5, 6, 7 oder 8 ist
      p 0, 1, 2, 3, 4, 5, 6, 7 oder 8 ist, Z' ausgewählt ist aus der Gruppe bestehend aus SH, NH₂ und alpha,beta-ungesättigten Carbonylgruppen und umsetzbar ist mit einer Gruppe Y unter Ausbildung zumindest einer kovalenten Bindung,
      umfassend in oder als Verbindung umfassend die Struktureinheit (IIIa) eine, zwei, drei, vier oder mehr Verbindungen ausgewählt aus Verbindungen gemäß Formel (VII) 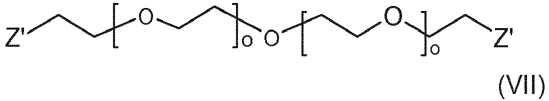 wobei
      o in jeder der zwei Seitenketten unabhängig von der Bedeutung von o in den jeweils anderen Seitenketten eine ganze Zahl größer oder gleich 1 ist und
      Z' jeder der zwei Seitenketten unabhängig von der Bedeutung von Z' in den jeweils anderen Seitenketten ausgewählt ist aus der Gruppe bestehend aus SH, NH₂ und alpha,beta-ungesättigten Carbonylgruppen und umsetzbar ist mit einer Gruppe Y unter Ausbildung zumindest einer kovalenten Bindung.

Unter einem Klebstoff wird im Rahmen dieses Textes eine Stoffmischung verstanden, die durch chemische Reaktion ihrer Bestandteile und/oder durch Einwirkung äußerer Stoffe abbindet und damit Werkstoffe und/oder Gewebe durch Oberflächenhaftung (Adhäsion) so verbinden kann, dass die Verbindung eine ausreichende innere Festigkeit (Kohäsion) besitzt.

Unter einem "Mehrkomponentenklebstoff" wird generell ein Klebstoff verstanden, der aus zwei oder mehr voneinander räumlich getrennten, reaktionsfähigen Komponenten besteht, die zur Umsetzung gemischt und erforderlichenfalls noch weiter behandelt werden. Die Bestandteile (z. B. eine Verbindung der Formel (I)) des erfindungsgemäßen Mehrkomponentenklebstoffes sind somit auf zwei, drei oder mehr getrennte Komponenten aufgeteilt, wobei vorzugsweise Bestandteile, bei denen es sich bei X, Y, Z oder Z' um SH und/oder NH₂ handelt, von Bestandteilen separiert sind, bei denen es sich bei X, Y, Z oder Z' um eine alpha,beta-ungesättigte Carbonylgruppe handelt. In bestimmten Ausführungsformen erfindungsgemäßer Mehrkomponentenklebstoffe, z. B. wenn eine Separation von zur Umsetzung miteinander vorgesehenen Bestandteilen, bei denen es sich bei X, Y, Z oder Z' um SH und/oder NH₂ handelt, von Bestandteilen, bei denen es sich bei X, Y, Z oder Z' um eine alpha,beta-ungesättigte Carbonylgruppe handelt, nicht möglich oder zweckdienlich ist, werden die weiteren Bestandteile des Mehrkomponentenklebstoffes so aufgeteilt, dass trotzdem eine Ausbildung des Adhäsivhydrogels (durch Abbinden) nicht vor dem Vermischen der Komponenten erfolgt. Dies kann beispielsweise dadurch geschehen, dass der pH-Wert einzelner oder aller Komponenten des Mehrkomponentenklebstoffs so eingestellt wird, dass eine Ausbildung des Adhäsivhydrogels erst nach dem Mischen der Komponenten oder nach dem Applizieren des Mehrkomponentenklebstoffes in ein entsprechendes Milieu erfolgt, das einen entsprechenden pH-Wert aufweist (z. B. Mundraum).

Unter "Hydrogelen" werden im Rahmen dieses Textes Gele auf Basis hydrophiler, aber wasserunlöslicher Polymere verstanden, die als dreidimensionale Netzwerke vorliegen. Durch den hydrophilen Charakter der dreidimensionalen Netzwerke sind Hydrogele in der Lage, Wasser aufzunehmen. Vorzugsweise enthalten die Hydrogele Wasser. Im Rahmen dieses Textes werden jedoch auch getrocknete Hydrogele, die kein oder nur Spuren von Wasser enthalten, als Hydrogel verstanden, sofern sie in der Lage sind, (zusätzliches) Wasser aufzunehmen.

Unter einem "Adhäsivhydrogel" wird ein Hydrogel verstanden, das sowohl die Eigenschaften eines Hydrogels aufweist als auch Werkstoffe und/oder Gewebe durch Oberflächenhaftung (Adhäsion) so verbinden kann, dass die Verbindung eine ausreichende innere Festigkeit (Kohäsion) besitzt. Nach dem Vermischen der Komponenten eines erfindungsgemäßen Mehrkomponentenklebstoffes entsteht durch chemische Reaktion der Bestandteile der einzelnen Komponenten ein Adhäsivhydrogel.

Offenbart wird auch ein Mehrkomponentenklebstoff zur Herstellung eines Adhäsivhydrogels
a) eine Verbindung der Formel (I) 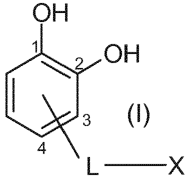 wobei
   X ausgewählt ist aus der Gruppe bestehend aus SH, NH₂ und alpha,beta-ungesättigten Carbonylgruppen und
   L ein Linker ist, der die 1,2-Dihydroxyphenyl-Einheit mit X verknüpft, wobei der Linker L in 3- oder 4-Position mit der 1,2-Dihydroxyphenyl-Einheit verbunden ist, und
b) eine, zwei, drei, vier oder mehr Verbindungen,
   jeweils enthaltend eine, zwei, drei, vier, fünf, sechs, sieben, acht oder mehr Struktureinheiten (II)

   B-L'-Y (II)

   wobei in jeder der Struktureinheiten (II) unabhängig von der Bedeutung in anderen Struktureinheiten (II)
   B ein Verknüpfungspunkt ist,
   L' ein Linker ist und
   Y ausgewählt ist aus der Gruppe bestehend aus SH, NH₂ und alpha,beta-ungesättigten Carbonylgruppen und umsetzbar ist mit der Gruppe X unter Ausbildung zumindest einer kovalenten Bindung,
   wobei
   (i) eine, zwei, drei, vier oder mehr der Verbindungen, die jeweils eine, zwei, drei, vier, fünf, sechs, sieben, acht oder mehrere Struktureinheiten (II) enthalten, zusätzlich jeweils einen Rest Z umfassen, der unabhängig von jedem anderen Rest Z einer anderen Verbindung ausgewählt ist aus der Gruppe bestehend aus SH, NH₂ und alpha,beta-ungesättigten Carbonylgruppen und umsetzbar ist mit einer Gruppe Y unter Ausbildung zumindest einer kovalenten Bindung.

Offenbart wird auch ein Mehrkomponentenklebstoff zur Herstellung eines Adhäsivhydrogels
a) eine Verbindung der Formel (I) 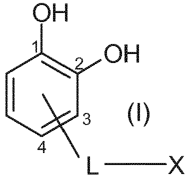 wobei
   X ausgewählt ist aus der Gruppe bestehend aus SH, NH₂ und alpha,beta-ungesättigten Carbonylgruppen und
   L ein Linker ist, der die 1,2-Dihydroxyphenyl-Einheit mit X verknüpft, wobei der Linker L in 3- oder 4-Position mit der 1,2-Dihydroxyphenyl-Einheit verbunden ist,
   und
b) eine, zwei, drei, vier oder mehr Verbindungen,
   jeweils enthaltend eine, zwei, drei, vier, fünf, sechs, sieben, acht oder mehr Struktureinheiten (II)

   B-L'-Y (II)

   wobei in jeder der Struktureinheiten (II) unabhängig von der Bedeutung in anderen Struktureinheiten (II)
   B ein Verknüpfungspunkt ist,
   L' ein Linker ist und
   Y ausgewählt ist aus der Gruppe bestehend aus SH, NH₂ und alpha,beta-ungesättigten Carbonylgruppen und umsetzbar ist mit der Gruppe X unter Ausbildung zumindest einer kovalenten Bindung,
und
(ii) zusätzlich eine, zwei, drei, vier oder mehr Verbindungen, die unabhängig voneinander jeweils zwei, drei, vier, fünf, sechs, sieben, acht oder mehr Struktureinheiten (III)

   B'-L"-Z' (III)

   umfassen, wobei in jeder der Struktureinheiten (III) unabhängig von der Bedeutung in anderen Struktureinheiten (III)
   B' ein Verknüpfungspunkt ist,
   L" ein Linker ist und
   Z' ausgewählt ist aus der Gruppe bestehend aus SH, NH₂ und alpha,beta-ungesättigten Carbonylgruppen und umsetzbar ist mit einer Gruppe Y unter Ausbildung zumindest einer kovalenten Bindung.

Es hat sich herausgestellt, dass erfindungsgemäße Mehrkomponentenklebstoffe besonders gute Eigenschaften aufweisen, wenn zumindest eine Komponente des Mehrkomponentenklebstoffes einen spezifischen pH-Wert aufweist. Nach Vermischen der Komponenten zum Zwecke der Ausbildung eines Adhäsivhydrogels können insbesondere bei der bevorzugten Anwesenheit von SH-Gruppen (Y, X und/oder Z' bedeuten dann SH in der jeweils entsprechenden Verbindung bzw. Struktureinheit der Formel (I), (II) oder (III)) bei einem pH-Wert von > 8 Nebenreaktionen auftreten, die die Herstellung des Adhäsivhydrogels negativ beeinflussen können. Bei einem pH-Wert von < 6 wird die Reaktionsgeschwindigkeit des durch Vermischen der Komponenten erhaltenen Reaktionsgemisches bei der Ausbildung des Adhäsivhydrogels häufig abgesenkt, so dass die Ausbildung des Adhäsivhydrogels verlangsamt wird. Bevorzugt ist daher ein erfindungsgemäßer Mehrkomponentenklebstoff, bei dem eine, zwei, drei oder sämtliche Komponenten einen pH-Wert im Bereich von 6 bis 8 aufweisen, bevorzugt im Bereich von 7 bis 8, besonders bevorzugt im Bereich von 7,5 bis 7,8.

Vorzugsweise sind die Komponenten des erfindungsgemäßen Mehrkomponentenklebstoffes so ausgestaltet, dass nach dem Zusammenmischen sämtlicher Komponenten in der resultierenden Reaktionsmischung ein pH-Wert im Bereich von 6 bis 8 vorliegt, bevorzugt im Bereich von 7 bis 8, besonders bevorzugt im Bereich von 7,5 bis 7,8. Dies gilt insbesondere bei der bevorzugten Anwesenheit von SH-Gruppen (Y, X und/oder Z' bedeuten dann SH in der jeweils entsprechenden Verbindung bzw. Struktureinheit der Formel (I), (IIa) oder (IIIa)).

Ein solcher pH-Wert-Bereich ist physiologisch sehr gut verträglich und liegt beispielsweise üblicherweise im Mundraum vor. Deshalb weisen die bevorzugten erfindungsgemäßen Mehrkomponentenklebstoffe eine besonders gute Biokompatibilität auf. Zudem verläuft die Ausbildung eines Adhäsivhydrogels in diesem pH-Wert-Bereich besonders gut, insbesondere bei der bevorzugten Anwesenheit von SH-Gruppen.

In einigen Ausführungsformen hat es sich als vorteilhaft erwiesen, den pH-Bereich, bzw. - Wert des aus einem erfindungsgemäßen Mehrkomponentenklebstoff resultierenden Reaktionsgemisches durch Zugabe eines Puffersystems zu stabilisieren. Bevorzugt ist daher ein erfindungsgemäßer Mehrkomponentenklebstoff umfassend c) einen Puffer bestehend aus einer Puffersäure und einer korrespondierenden Pufferbase, wobei der pK_{B} der Pufferbase nicht kleiner ist als 2, vorzugsweise nicht kleiner ist als 4.

Die Verwendung eines Puffersystems führt zu einer Stabilisierung des pH-Bereiches bzw. -Wertes im Reaktionsgemisch eines erfindungsgemäßen Mehrkomponentenklebstoffes. Dadurch können beispielsweise mögliche Nebenreaktionen vermieden werden, die ansonsten während der Ausbildung eines Adhäsivhydrogels bei pH-Werten von > 8 auftreten können. Dies gilt insbesondere bei der bevorzugten Anwesenheit von SH-Gruppen (Y, X und/oder Z' bedeuten dann SH in der jeweils entsprechenden Verbindung bzw. Struktureinheit der Formel (I), (IIa) oder (IIIa)). Zudem lassen sich durch Einsatz eines Puffersystems die pH-Werte der Komponenten und der pH-Wert der durch Vermischen aller Komponenten resultierenden Mischung sehr genau einstellen.

Bevorzugt ist insbesondere ein erfindungsgemäßer Mehrkomponentenklebstoff, bei dem das molare Verhältnis von Puffersäure zu Pufferbase im Bereich von 1:10 bis 10:1 liegt, bevorzugt im Bereich von 1:8 bis 8:1, besonders bevorzugt im Bereich von 1:5 bis 5:1. In diesem Bereich des molaren Verhältnisses von Puffersäure zur Pufferbase weist der Puffer regelmäßig eine ausreichende Pufferkapazität auf.

Dem Fachmann sind zahlreiche Puffersysteme bekannt, die auf einen gewünschten pH-Wert im Bereich zwischen 6 und 8 eingestellt werden können.

In eigenen Untersuchungen hat es sich gezeigt, dass Puffersysteme, die primäre Amin-Gruppen und/oder Puffersysteme, die 3-(*N*-Morpholino)propansulfonsäure (MOPS) enthalten, die Ausbildung eines erfindungsgemäßen Adhäsivhydrogels aus dem erfindungsgemäßen Mehrkomponentenklebstoff in manchen Fällen in relevantem Maße behindern; die Anwesenheit von Sulfonsäure-Gruppen erscheint insgesamt nicht bevorzugt. Wahrscheinlich finden Nebenreaktionen zwischen den Amin-Gruppen und/oder Sulfonsäure-Gruppen des Puffers und den zur Reaktion miteinander vorgesehenen weiteren Bestandteilen des Mehrkomponentenklebstoffes statt. Entsprechend ist ein erfindungsgemäßer Mehrkomponentenklebstoff besonders bevorzugt, bei dem die Puffersäure und/oder Pufferbase keine die Ausbildung eines Adhäsivhydrogels störenden chemischen Reaktionen mit den Bestandteilen des Mehrkomponentenklebstoffes eingehen. Insbesondere ist ein erfindungsgemäßer Mehrkomponentenklebstoff besonders bevorzugt, bei dem die Puffersäure und/oder Pufferbase keine Amin-Gruppen und/oder Sulfonsäure-Gruppen enthält.

Bei eigenen Untersuchungen konnten zahlreiche Puffersysteme identifiziert werden, deren pH-Wert im Bereich zwischen 6 und 8 eingestellt werden kann und die keine unerwünschten Wechselwirkungen (z. B. chemische Reaktionen) mit den Bestandteilen des erfindungsgemäßen Mehrkomponentenklebstoffs eingehen. Bevorzugt ist daher ein erfindungsgemäßer Mehrkomponentenklebstoff, umfassend einen oder mehrere Puffer (Puffersysteme) ausgewählt aus der Gruppe bestehend aus Maleat-Puffer, Citrat-Puffer, Bis(2-hydroxyethyl)amino-tris(hydroxymethyl)methan-Puffer (Bis-Tris Puffer), PhosphatPuffer, N-(2-Acetamido)-iminodiessigsäure,(ADA) Carbonat-Puffer, Imidazol-Puffer, 1,3-Bis-[tris-(hydroxymethyl)-methylamino]propan-Puffer (Bis-Tris-Propan Puffer), Triethanolamin-Puffer, Tricin-Puffer und Bicin-Puffer.

Die Ausbildung eines erfindungsgemäßen Adhäsivhydrogels erfolgt durch chemische Reaktion der Bestandteile des erfindungsgemäßen Mehrkomponentenklebstoffs. Dabei reagieren vorzugsweise die SH- und/oder NH₂-Gruppen mit den alpha,beta-ungesättigten Carbonylgruppen im Rahmen einer Michael-Addition. Die SH- und/oder NH₂-Gruppen agieren dabei als Michael-Donatoren und die alpha,beta-ungesättigten Carbonylgruppen als Michael-Akzeptoren. Diese Michael-Addition verläuft bei Anwesenheit einer starken Base besonders gut. Bevorzugt ist daher ein erfindungsgemäßer Mehrkomponentenklebstoff, der als Bestandteil d) eine oder mehrere Basen mit einem pK_{B} < 1 umfasst.

Besonders bevorzugt ist ein solcher erfindungsgemäßer Mehrkomponentenklebstoff, bei dem es sich bei der einen oder den Basen mit einem pK_{B} < 1 um Ammoniak, Ammoniumhydroxid und/oder Hydroxide der Alkalimetalle oder Erdalkalimetalle handelt, vorzugsweise um Natriumhydroxid oder Kaliumhydroxid. Die resultierenden erfindungsgemäßen Mehrkomponentenklebstoffe weisen eine sehr gute Biokompatibilität auf.

### Bestandteil a), Verbindung der Formel (I):

Die Verbindung der Formel (I) in einem erfindungsgemäßen Mehrkomponentenklebstoff ist für eine hervorragende Adhäsion des aus dem erfindungsgemäßen Mehrkomponentenklebstoff resultierenden Adhäsivhydrogels mitverantwortlich. Die 1,2-Dihydroxyphenyl-Einheit der Verbindung der Formel (I) ist in der Lage, starke Adhäsionskräfte zu anderen Oberflächen (z. B. biologischen Geweben oder Titanoberflächen) auszubilden. In eigenen Untersuchungen hat es sich gezeigt, dass der Linker L in der Verbindung der Formel (I) einen Einfluss auf die Eigenschaften des Mehrkomponentenklebstoffs bzw. des daraus resultierenden Adhäsivhydrogels besitzt (z. B. Biokompatibilität, biologische Abbaubarkeit, gute Adhäsion an biologischem Gewebe allogenen, heterogenen oder synthetischen Ursprungs und/oder an medizinischen Implantaten).

Im erfindungsgemäßen Mehrkomponentenklebstoff hat es sich als vorteilhaft erwiesen, den Linker L in einer bestimmten Länge vorzusehen. Im erfindungsgemäßen Mehrkomponentenklebstoff, ist die Anzahl der Kettenglieder des Linkers L im Bereich von 5 bis 65, vorzugsweise 6 bis 60, bevorzugt 24 bis 36. Es hat sich in eigenen Untersuchungen gezeigt, dass die aus solchen erfindungsgemäßen Mehrkomponentenklebstoffen resultierenden Adhäsivhydrogele eine gute Adhäsion an biologischem Gewebe allogenen, heterogenen oder synthetischen Ursprungs und/oder an medizinischen Implantaten aufweisen.

Unter einem Kettenglied des Linkers L wird im Rahmen des vorliegenden Textes ein einzelnes Atom (Kettenatom oder Kettenglied) verstanden, das in Kombination mit weiteren durch kovalente Bindungen aneinandergereihten Atomen (weitere Kettenatome oder Kettenglieder) die 1,2-Dihydroxyphenyl-Einheit der Verbindung der Formel (I) auf kürzestem Weg mit der funktionellen Gruppe X verknüpft. Es werden nur die Atome als Kettenglieder gezählt, die auf dem kürzesten Weg zwischen der 1,2-Dihydroxyphenyl-Einheit der Verbindung der Formel (I) und X liegen; Atome in Seitenketten werden nicht berück sichtigt. Für den Fall, dass eine Verbindung der Formel (I) sowohl eine oder mehrere SH-Gruppen als auch eine oder mehrere NH₂-Gruppen umfasst, wird (werden) nur die SH-Gruppe(n) berücksichtigt. Für den Fall, dass eine Verbindung der Formel (I) zwei oder mehr SH-Gruppen oder (bei Abwesenheit von SH-Gruppen) NH₂-Gruppen umfasst, wird die Gruppe SH bzw. NH₂ als X betrachtet, die auf kürzestem Weg mit der 1,2-Dihydroxyphenyl-Einheit und der SH oder NH₂-Gruppe verknüpft ist. Beispielsweise beträgt die Anzahl der Kettenglieder des Linkers L in der nachfolgenden Verbindung der Formel (IV) 30 (dies ist die Anzahl der Atome zwischen der Gruppe SH und der 1,2-Dihydroxyphenyl-Einheit).

Der Linker L kann dabei auf vielfache Art und Weise ausgestaltet sein. Ein erfindungsgemäßer Mehrkomponentenklebstoff ist ein solcher bei dem der Linker L ein Peptid ist oder eine solche Gruppe umfasst. Die aus solchen erfindungsgemäßen Mehrkomponentenklebstoffen resultierenden Adhäsivhydrogele weisen eine sehr gute Biokompatibilität auf.

Um die Haftung (Adhäsion) des aus dem erfindungsgemäßen Mehrkomponentenklebstoff hergestellten Adhäsivhydrogels zu verbessern, hat es sich als vorteilhaft erwiesen, wenn in der Verbindung der Formel (I) neben der 1,2-Dihydroxyphenyl-Einheit auch Alkylamine vorhanden sind. Dementsprechend ist ein erfindungsgemäßer Mehrkomponentenklebstoff bevorzugt, wobei der Linker L ein, zwei, drei, vier oder mehr Alkylamine als Seitenkette enthält.

Offenbart ist auch ein Mehrkomponentenklebstoff, wobei die Verbindung der Formel (I) eine oder mehrere Struktureinheiten (IX) enthält 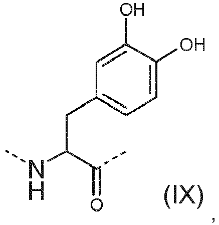 wobei zumindest eine der gestrichelten Linien eine kovalente Bindung zu weiteren Struktureinheiten darstellt. Für den Fall, dass die Struktureinheit (IX) terminal in einer Verbindung der Formel (I) vorliegt, entspricht eine der gestrichelten Linien Wasserstoff oder - OH, abhängig davon ob die Struktureinheiten (IX) über die Carbonsäurefunktion oder über die Amin-Funktion mit weiteren Struktureinheiten kovalent verbunden ist.

Bei einigen Anwendungen hat sich ein Mehrkomponentenklebstoff als besonders geeignet herausgestellt, in dem die Verbindung der Formel (I) spezifische Aminosäureeinheiten bzw. Kombinationen von spezifischen Aminosäureeinheiten enthält. Besonders bevorzugt ist dabei ein Mehrkomponentenklebstoff, wobei die Verbindung der Formel (I) zumindest eine Cystein-Einheit, vorzugsweise eine L-Cystein-Einheit (Formel X) enthält.

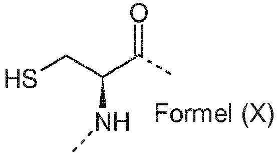

Zumindest eine der gestrichelten Linien in Formel (X) stellt eine kovalente Bindung zu weiteren Struktureinheiten da. Für den Fall, dass die Cystein-Einheit terminal in der Verbindung der Formel (I) vorliegt, entspricht eine der gestrichelten Linien Wasserstoff oder -OH, abhängig davon ob die Cystein-Einheit über die Carbonsäurefunktion oder über die Amin-Funktion mit weiteren Struktureinheiten kovalent verbunden ist. Beispielsweise ist die Cystein-Einheit in der nachfolgenden Verbindung der Formel (IV) terminal angeordnet, über die Carbonsäurefunktion (unter Ausbildung eines Amids) mit weiteren Struktureinheiten kovalent verbunden und die Amin-Funktion der Cystein-Einheit liegt als NH₂ vor.

Bei der Herstellung eines erfindungsgemäßen Mehrkomponentenklebstoffes und der Herstellung eines erfindungsgemäßen Adhäsivhydrogels (siehe auch Beispiele 2 und 3) ermöglicht die Anwesenheit einer Thiolgruppe (SH-Gruppe) in der Verbindung der Formel (I) die Michael-Addition bei einem pH-Wert auf physilogischem Niveau (pH 6 bis 7,5); bei einem solchen pH-Wert wird gleichzeitig die Autooxidation der Dihydroxygruppe verhindert.
Mehrkomponentenklebstoffe, in denen die Verbindung der Formel (I) spezifische Aminosäureeinheiten bzw. Kombinationen von bestimmten Aminosäureeinheiten enthält, zeichnen sich durch eine besonders gute Biokompatibilität aus und/oder sind biologisch abbaubar. Darüber hinaus kann in den Fällen, in denen die Verbindung der Formel (I) spezifische Aminosäureeinheiten bzw. Kombinationen von bestimmten Aminosäureeinheiten enthält, das Adhäsivhydrogel aus dem Mehrkomponenten Klebstoff durch Quervernetzung ohne Zugabe eines Oxidationsmittels oder Enzym hergestellt werden. Bevorzugt ist ein Mehrkomponentenklebstoff, wobei der Linker L in der Verbindung der Formel (I) ein, zwei oder mehr Struktureinheiten enthält, ausgewählt aus der Gruppe bestehend aus Glycin, Alanin, Valin, Leucin, Isoleucin, Methionin, Phenylalanin, Tryptophan, Prolin, Serin, Threonin, Cystein, Tyrosin, Asparagin, Glutamin, Asparaginsäure, Glutaminsäure, Lysin, Arginin, Histidin, vorzugsweise ausgewählt aus der Gruppe bestehend aus Cystein, Alanin, Lysin, Prolin, Serin, Tyrosin und Threonin.

Erfindungsgemäß ist ein Mehrkomponentenklebstoff, bei dem der Linker L in der Verbindung der Formel (I) ein, zwei oder mehr Struktureinheiten enthält, ausgewählt aus der Gruppe von Aminosäuresequenzen bestehend aus Alanin-Lysin, Lysin-Prolin, Prolin-Serin, Serin-Tyrosin, Tyrosin-Prolin, Prolin-Prolin, Prolin-Threonin, Alanin-Lysin-Prolin, Lysin-Prolin-Serin, Prolin-Serin-Tyrosin, Serin-Tyrosin-Prolin, Tyrosin-Prolin-Prolin, Prolin-Prolin-Threonin, Alanin-Lysin-Prolin-Serin (SEQ ID NO: 1), Lysin-Prolin-Serin-Tyrosin (SEQ ID NO: 2), Prolin-Serin-Tyrosin-Prolin (SEQ ID NO: 3), Serin-Tyrosin-Prolin-Prolin (SEQ ID NO: 4), Tyrosin-Prolin-Prolin-Threonin (SEQ ID NO: 5), Alanin-Lysin-Prolin-Serin-Tyrosin (SEQ ID NO: 6), Lysin-Prolin-Serin-Tyrosin-Prolin (SEQ ID NO: 7), Prolin-Serin-Tyrosin-Prolin-Prolin (SEQ ID NO: 8), Serin-Tyrosin-Prolin-Prolin-Threonin (SEQ ID NO: 9), Alanin-Lysin-Prolin-Serin-Tyrosin-Prolin (SEQ ID NO: 10), Lysin-Prolin-Serin-Tyrosin-Prolin-Prolin (SEQ ID NO: 11), Prolin-Serin-Tyrosin-Prolin-Prolin-Threonin (SEQ ID NO: 12), Alanin-Lysin-Prolin-Serin-Tyrosin-Prolin-Prolin (SEQ ID NO: 13), Lysin-Prolin-Serin-Tyrosin-Prolin-Prolin-Threonin (SEQ ID NO: 14) und Alanin-Lysin-Prolin-Serin-Tyrosin-Prolin-Prolin-Threonin (SEQ ID NO: 15).

Die "SEQ ID NO" beziehen sich auf das beigefügte Sequenzprotokoll, das Bestandteil der vorliegenden Beschreibung ist.

Ein erfindungsgemäßer Mehrkomponentenklebstoff ist besonders bevorzugt, in dem die Verbindung der Formel (I) die Struktur gemäß Formel (IV) (SEQ ID NO: 16) besitzt:

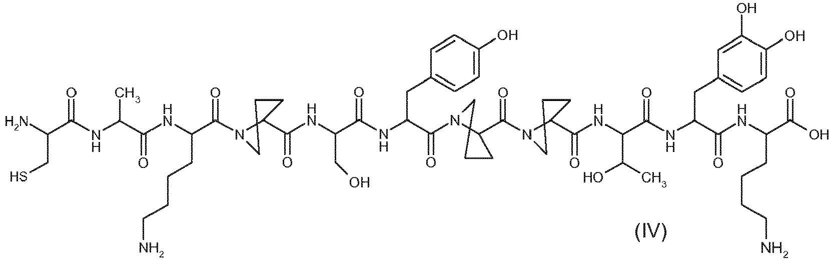

Das aus einem die Verbindung der Formel (IV) enthaltenden erfindungsgemäßen Mehrkomponentenklebstoff resultierende erfindungsgemäße Adhäsivhydrogel weist eine sehr gute Adhäsion an biologischem Gewebe allogenen, heterogenen oder synthetischen Ursprungs und/oder an medizinischen Implantaten, eine gute Biokompatibilität und eine gute biologische Abbaubarkeit auf.

Bei der Verbindung der Formel (IV) handelt es sich um ein bevorzugtes Beispiel einer Verbindung der Formel (I).

Bevorzugt ist ein erfindungsgemäßer Mehrkomponentenklebstoff, bei dem, sofern X in der Verbindung der Formel (I) eine alpha,beta-ungesättigte Carbonylgruppe bedeutet, X ausgewählt ist aus der Gruppe bestehend aus alpha,beta-ungesättigten Aldehyden, alpha,beta-ungesättigten Ketonen, alpha,beta-ungesättigten Estern, vorzugsweise Acrylsäureester, und alpha,beta-ungesättigten Carbonsäureamiden, vorzugsweise Maleinimid.

### Bestandteil b), Verbindung enthaltend die Struktureinheit (IIa):

Im erfindungsgemäßen Mehrkomponentenklebstoff besitzt die Struktureinheit (IIa) die nachfolgende Struktur: 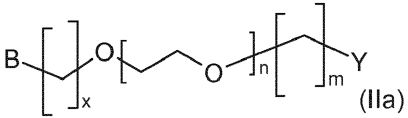 wobei n eine ganze Zahl größer oder gleich 1 ist, x 0, 1, 2, 3, 4, 5, 6, 7, oder 8 ist und m 0, 1, 2, 3, 4, 5, 6, 7, oder 8 ist. Die aus solchen erfindungsgemäßen Mehrkomponentenklebstoffen resultierenden Adhäsivhydrogele, weisen eine sehr gutes Wasseraufnahmevermögen und eine sehr gute Biokompatibilität auf.

Der erfindungsgemäße Mehrkomponentenklebstoff umfasset in oder als Bestandteil (b) eine, zwei, drei, vier oder mehr Verbindungen ausgewählt aus Verbindungen der Formel (V) 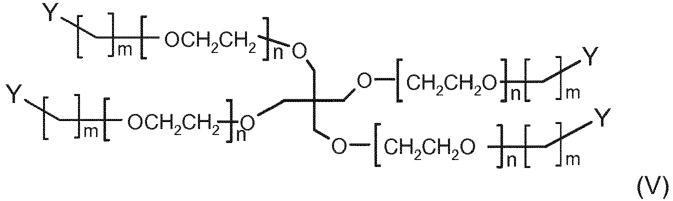 wobei
n in jeder der vier Seitenketten unabhängig von der Bedeutung von n in den jeweils anderen Seitenketten eine ganze Zahl größer oder gleich 1 ist und
m in jeder der vier Seitenketten unabhängig von der Bedeutung von m in den jeweils anderen Seitenketten 0, 1, 2, 3, 4, 5, 6, 7, oder 8 ist
   und
Y in jeder der vier Seitenketten unabhängig von der Bedeutung von Y in den jeweils anderen Seitenketten ausgewählt ist aus der Gruppe bestehend aus SH, NH₂ und alpha,beta-ungesättigten Carbonylgruppen und umsetzbar ist mit der Gruppe X unter Ausbildung zumindest einer kovalenten Bindung.

Ganz besonders bevorzugt ist ein erfindungsgemäßer Mehrkomponentenklebstoff wie soeben definiert, wobei in der Verbindung der Formel (V)
m in jeder der vier Seitenketten identisch ist
und/oder
Y in jeder der vier Seitenketten identisch ist.

Da die Verbindung der Formel (V) in den besonders bevorzugten Ausgestaltungen eine hohe Symmetrie aufweist und alle Seitenarme (als Beispiel für Struktureinheiten (IIa)) identisch sind, lässt sich diese Verbindung besonders einfach herstellen. Dadurch lassen sich beispielsweise die Kosten der Verbindung mit der Formel (V) und somit auch die Kosten eines erfindungsgemäßen Mehrkomponentenklebstoffes reduzieren. Durch den einfachen synthetischen Zugang zu Verbindungen der Formel (V) in wenigen Reaktionsschritten lässt sich die Verbindung der Formel (V) auch in hoher Reinheit erhalten. Verunreinigungen und Nebenprodukte, die bei Reaktionen mit vielen Syntheseschritten zwangläufig entstehen würden und nicht abgetrennt werden können, werden vermieden. Dadurch kann ein erfindungsgemäßer Mehrkomponentenklebstoff in sehr guter Reinheit und Qualität erhalten werden.

Insbesondere bevorzugt ist ein erfindungsgemäßer Mehrkomponentenklebstoff wie soeben definiert, umfassend in oder als Bestandteil (b) eine Verbindung der Formel (VI) 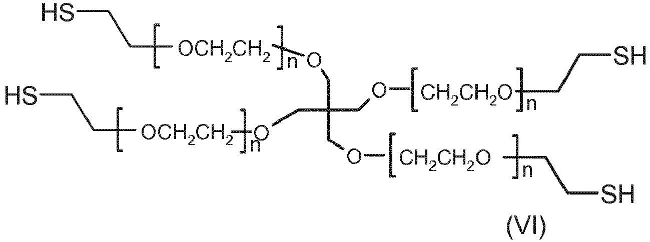 wobei
n in jeder der vier Seitenketten unabhängig von der Bedeutung von n in den jeweils anderen Seitenketten eine ganze Zahl größer oder gleich 1 ist.

Bei der Verbindung der Formel (VI) handelt es sich um ein besonders bevorzugtes Beispiel einer Verbindung der Formel (V) und eine besonders bevorzugte Ausgestaltung einer Verbindung enthaltend die Struktureinheit (IIa).

Die SH-Gruppen der Verbindung der Formel (VI) (und ähnlicher Verbindungen) reagieren sehr selektiv, mit hohen Ausbeuten und unter milden Bedingungen mit alpha,beta-ungesättigten Carbonylgruppen. Somit weisen die entsprechenden erfindungsgemäßen Mehrkomponentenklebstoffe und die daraus entstehenden erfindungsgemäßen Adhäsivhydrogele eine sehr hohe Qualität auf.

Besonders bevorzugt ist ein erfindungsgemäßer Mehrkomponentenklebstoff, wobei eine, zwei, drei, vier oder sämtliche Verbindungen, die unabhängig voneinander jeweils zwei, drei, vier, fünf, sechs, sieben, acht oder mehr Struktureinheiten (II) umfassen, (a) ein Gewichtsmittel der Molmasse (M_{w}) im Bereich von 500 bis 40000 g/mol aufweisen, bevorzugt im Bereich von 500 bis 25000 g/mol, besonders bevorzugt im Bereich von 1000 bis 15000 g/mol, insbesondere bevorzugt im Bereich von 2000 bis 8000 g/mol und/oder (b) eine Polydispersität von kleiner als 4 aufweisen, bevorzugt kleiner als 3, besonders bevorzugt kleiner als 2, insbesondere bevorzugt kleiner als 1,5.

### Bestanteil (ii), Verbindung enthaltend die Struktureinheit (IIIa):

Im erfindungsgemäßen Mehrkomponentenklebstoff besitzt die Struktureinheit (IIIa) die nachfolgende Struktur: 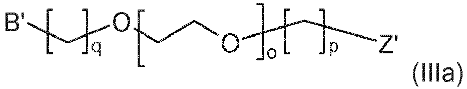 wobei o eine ganze Zahl größer oder gleich 1 ist, q 0, 1, 2, 3, 4, 5, 6, 7, oder 8 ist und p 0, 1, 2, 3, 4, 5, 6, 7, oder 8 ist. Die aus diesen erfindungsgemäßen Mehrkomponentenklebstoffen resultierenden Adhäsivhydrogele, weisen eine sehr gutes Wasseraufnahmevermögen und eine sehr gute Biokompatibilität auf.

Besonders bevorzugt ist ein erfindungsgemäßer Mehrkomponentenklebstoff, umfassend in oder als Verbindung umfassend die Struktureinheit (IIIa) eine, zwei, drei, vier oder mehr Verbindungen ausgewählt aus Verbindungen gemäß Formel (VII) 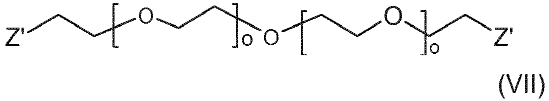
wobei o in jeder der zwei Seitenketten unabhängig von der Bedeutung von o in den jeweils anderen Seitenketten eine ganze Zahl größer oder gleich 1 ist und
Z' jeder der zwei Seitenketten unabhängig von der Bedeutung von Z' in den jeweils anderen Seitenketten ausgewählt ist aus der Gruppe bestehend aus SH, NH₂ und alpha,beta-ungesättigten Carbonylgruppen und umsetzbar ist mit einer Gruppe Y unter Ausbildung zumindest einer kovalenten Bindung.

Da die Verbindung der Formel (VII) eine hohe Symmetrie aufweist und beide Seitenarme (Struktureinheiten (IIIa)) identisch sind, lässt sich diese Verbindung besonders einfach herstellen. Dadurch lassen sich beispielsweise die Kosten der Verbindung mit der Formel (VII) und somit auch die Kosten eines erfindungsgemäßen Mehrkomponentenklebstoffes reduzieren. Durch den einfachen synthetischen Zugang zu Verbindungen der Formel (VII) in wenigen Reaktionsschritten, lässt sich die Verbindung der Formel (VII) auch in hoher Reinheit erhalten. Verunreinigungen und Nebenprodukte, die bei Reaktionen mit vielen Syntheseschritten zwangläufig entstehen würden und nicht abgetrennt werden können, werden vermieden. Dadurch kann ein erfindungsgemäßer Mehrkomponentenklebstoff in sehr guter Reinheit und Qualität erhalten werden.

Ganz besonders bevorzugt ist ein erfindungsgemäßer Mehrkomponentenklebstoff, umfassend in oder als Bestandteil (ii) eine Verbindung der Formel (VIII) 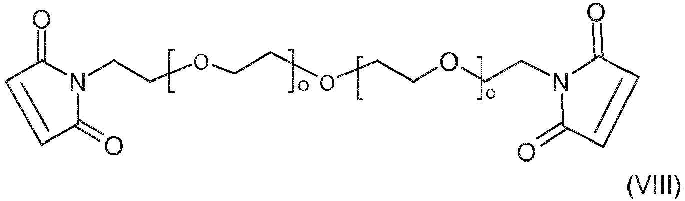 wobei o eine ganze Zahl größer oder gleich 1 ist.

Bei der Verbindung der Formel (VIII) handelt es sich um ein besonders bevorzugtes Beispiel einer Verbindung der Formel (VII) und eine besonders bevorzugte Ausgestaltung einer Verbindung enthaltend die Struktureinheit B'-L"-Z' (III) bzw. die Struktureinheit (IIIa).

Die alpha,beta-ungesättigten Carbonylgruppen der Verbindung mit der Formel (VIII) sind Maleinimidgruppen. Diese Maleinimidgruppen reagieren sehr selektiv, mit hohen Ausbeuten und unter milden Bedingungen mit Michael-Donatoren wie SH und NH₂, insbesondere mit SH. Entsprechend weisen die resultierenden erfindungsgemäßen Mehrkomponentenklebstoffe und die daraus entstehenden erfindungsgemäßen Adhäsivhydrogele eine sehr hohe Qualität auf.

Besonders bevorzugt ist ein erfindungsgemäßer Mehrkomponentenklebstoff, wobei eine, zwei, drei, vier oder sämtliche Verbindungen, die unabhängig voneinander jeweils zwei, drei, vier, fünf, sechs, sieben, acht oder mehr Struktureinheiten (IIIa) umfassen, (a) ein Gewichtsmittel der Molmasse (M_{w}) im Bereich von 500 bis 40000 g/mol aufweisen, bevorzugt im Bereich von 500 bis 25000 g/mol, besonders bevorzugt im Bereich von 1000 bis 15000 g/mol, insbesondere bevorzugt im Bereich von 2000 bis 8000 g/mol und/oder (b) eine Polydispersität von kleiner als 4 aufweisen, bevorzugt kleiner als 3, besonders bevorzugt kleiner als 2, insbesondere bevorzugt kleiner als 1,5.

Das Gewichtsmittel der Molmasse (M_{w}) wird mittels Gel-Permeations-Chromatographie mit Polystyrol (PS) als Standard bestimmt. Die Polydispersität entspricht dem Quotienten aus dem Massenmittel der Molmassen (M_{w}) und dem Zahlenmittel der Molmasse (Mₙ).

Die einzelnen (insbesondere die bevorzugten) Ausgestaltungen (wie oben beschrieben) der oben im Detail diskutierten Bestandteile a), b), c), d) und (ii) eines erfindungsgemäßen Mehrkomponentenklebstoffe können untereinander kombiniert werden.

Besonders bevorzugt ist ein erfindungsgemäßer Mehrkomponentenklebstoff zur Herstellung eines Adhäsivhydrogels, umfassend
a) eine Verbindung der Formel (IV) (SEQ ID NO: 16) (als Beispiel einer Verbindung der Formel (I)) 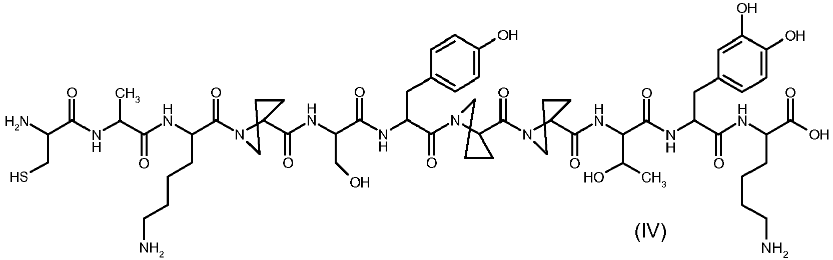 und
b) eine Verbindung der Formel (VI) (als Beispiel einer Verbindung der Formel (II)) 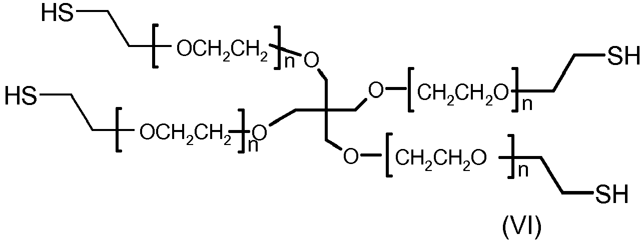 wobei
   n in jeder der vier Seitenketten unabhängig von der Bedeutung von n in den jeweils anderen Seitenketten eine ganze Zahl größer oder gleich 1 ist
   und
   eine Verbindung der Formel (VIII) (als Beispiel einer Verbindung der Formel (III)) 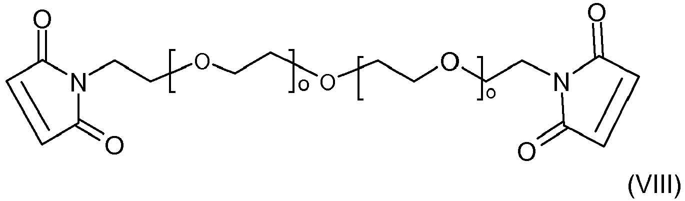 wobei o eine ganze Zahl größer oder gleich 1 ist
   und
c) einen Puffer bestehend aus einer Puffersäure und einer korrespondierenden Pufferbase, wobei der pK_{B} der Pufferbase nicht kleiner ist als 2, vorzugsweise nicht kleiner ist als 4
   und
d) eine oder mehrere Basen mit einem pK_{B} < 1,
wobei eine, zwei, drei oder sämtliche Komponenten des Mehrkomponentensystems einen pH-Wert im Bereich von 6 bis 8 aufweisen.

Bevorzugt ist ein erfindungsgemäßer Mehrkomponentenklebstoff, der biokompatibel und/oder bioabbaubar ist.

Unter dem Begriff "biokompatibel" wird in diesem Zusammenhang die Fähigkeit des Mehrkomponentenklebstoffes verstanden, keinen oder nur einen geringen negativen Einfluss auf lebendes Gewebe in der Umgebung des Mehrkomponentenklebstoffes aufzuweisen. "Biokompatibel" sind einerseits Mehrkomponentenklebstoffe, bei deren biologischer Beurteilung nach DIN EN ISO 10993-1:2010-04 keine chronische Toxizität, Kanzerogenität, Toxikokinetik, Immunotoxizität, Reproduktions-/Entwicklungstoxizität und andere organspezifischen Toxizitäten festgestellt wird, andererseits sonstige biokompatible Mehrkomponentenklebstoffe, insbesondere solche Mehrkomponentenklebstoffe, bei denen negative Wirkungen durch positive Wirkungen mindestens ausgeglichen werden.

Unter dem Begriff "bioabbaubar" wird in diesem Zusammenhang das Vermögen des Mehrkomponentenklebstoffes verstanden, biologisch abgebaut zu werden. "Bioabbaubar" sind einerseits "enzymatisch abbaubare", also durch Enzyme zersetzbare Mehrkomponentenklebstoffe, andererseits auf sonstige Weise biologisch abbaubare Mehrkomponentenklebstoffe, insbesondere auf biologischem Wege hydrolytisch abbaubare Mehrkomponentenklebstoffe.

Ein bevorzugter bioabbaubarer erfindungsgemäßer Mehrkomponentenklebstoff besitzt eine inhärente Abbaubarkeit (vorzugsweise gemäß Testmethode der Organisation für wirtschaftliche Zusammenarbeit und Entwicklung "OECD 302").

Bevorzugt ist ein erfindungsgemäßer Mehrkomponentenklebstoff zur Anwendung in einem Verfahren zur chirurgischen oder therapeutischen Behandlung des menschlichen oder tierischen Körpers.

Ebenfalls bevorzugt ist ein erfindungsgemäßer Mehrkomponentenklebstoff, zum Ankleben von (a) medizinischen und/oder (b) metallischen Implantaten, vorzugsweise medizinischen, metallischen Implantaten, die vorzugsweise einen Titangehalt von mehr als 89 Gew.-%, bezogen auf die Gesamtmasse des Implantates, aufweisen, an menschliches oder tierisches Gewebe, wobei das Gewebe vorzugsweise ausgewählt ist aus der Gruppe bestehend aus Knochen, Schleimhaut, Gingiva, Haut, Knorpel, Zahn, Parodont, Bindegewebe, Muskelgewebe, Nervengewebe und Epithelgewebe.

Ebenfalls bevorzugt ist ein erfindungsgemäßer Mehrkomponentenklebstoff zum Zusammenkleben von zwei Teilen aus menschlichem oder tierischem Gewebe, wobei (i) jedes der beiden Gewebe unabhängig von dem jeweils anderen Gewebe vorzugsweise ausgewählt ist aus der Gruppe bestehend aus Knochen, Schleimhaut, Gingiva, Haut, Knorpel, Zahnsubstanz, Parodont, Bindegewebe, Muskelgewebe, Nervengewebe und Epithelgewebe, und/oder (ii) die Gewebe allogen, heterogen oder zumindest ein Gewebe synthetisch oder biologisch herstellbar ist. Ein rein künstlich hergestelltes Gewebe ohne lebende Zusätze (z.B. Proteine) bzw. Ausgangsmaterialien (z.B. Zellen) ist ein synthetisches Gewebe; ein Herstellungsverfahren für Gewebe, bei dem lebende Zellen oder z.B. Bakterien eingesetzt werden, ist ein biologisches Herstellungsverfahren, das zu biologisch herstellbarem Gewebe führt.

Ebenfalls bevorzugt ist ein erfindungsgemäßer Mehrkomponentenklebstoff zum Zusammenkleben (a) eines Implantates mit Gingiva, (b) eines Implantates mit einem Biomaterial (allogen, heterogen oder synthetisch), (c) eines Implantates mit Knochen, (d) eines Biomaterials (allogen, heterogen oder synthetisch) mit Gingiva, (c) eines Biomaterials (allogen, heterogen oder synthetisch) mit Knochen, (d) eines Biomaterials (allogen, heterogen oder synthetisch) mit Zahnsubstanz, (e) eines Biomaterials (allogen, heterogen oder synthetisch) mit Parodont, (f) von Knochen mit Gingiva, (g) von Knochen mit Parodont, (h) von Zahnsubstanz mit Gingiva, (i) von Zahnsubstanz mit Parodont, (j) von Parodont mit Gingiva, (k) eines Implantates mit einem chemischen oder biologischen Behandlungsstoff, (l) eines Biomaterials (allogen, heterogen oder synthetisch) mit einem chemischen oder biologischen Behandlungsstoff, (m) von Knochen mit einem chemischen oder biologischen Behandlungsstoff, (n) von Zahnsubstanz mit einem chemischen oder biologischen Behandlungsstoff oder (o) von Parodont mit einem chemischen oder biologischen Behandlungsstoff.

Bevorzugt ist ein erfindungsgemäßer Mehrkomponentenklebstoff, umfassend die Verbindung der Formel (I) in einer Menge von 1 bis 15 Gew.-%, vorzugsweise in einer Menge von 3 bis 10 Gew.-%, bevorzugt in einer Menge von 4 bis 7 Gew.-%, bezogen auf die Gesamtmasse der Verbindung der Formel (I), der Verbindung(en), jeweils enthaltend eine, zwei, drei, vier, fünf, sechs, sieben, acht oder mehr Struktureinheiten (IIa), und der Verbindung(en), jeweils enthaltend eine, zwei, drei, vier, fünf, sechs, sieben, acht oder mehr Struktureinheiten (IIIa).

Besonders bevorzugt ist ein erfindungsgemäßer Mehrkomponentenklebstoff, umfassend
- die Verbindung der Formel (I), wobei die Gesamtmenge der Verbindung der Formel (I) im Bereich von 1 bis 15 Gew.-% liegt, vorzugsweise im Bereich von 3 bis 10 Gew.-%, bevorzugt im Bereich von 4 bis 7 Gew.-%, bezogen auf die Gesamtmasse der Verbindung der Formel (I), der Verbindung(en), jeweils enthaltend eine, zwei, drei, vier, fünf, sechs, sieben, acht oder mehr Struktureinheiten (IIa), und der Verbindung(en), jeweils enthaltend eine, zwei, drei, vier, fünf, sechs, sieben, acht oder mehr Struktureinheiten (IIIa),
- eine, zwei, drei, vier oder mehr Verbindungen, jeweils enthaltend eine, zwei, drei, vier, fünf, sechs, sieben, acht oder mehr Struktureinheiten (IIa), wobei die Gesamtmenge der Verbindungen enthaltend eine, zwei, drei, vier oder mehr Struktureinheiten (IIa) im Bereich von 55 bis 70 Gew.-% liegt, vorzugsweise im Bereich von 60 bis 67 Gew.-%, bevorzugt im Bereich von 63 bis 66 Gew.-%, bezogen auf die Gesamtmasse der Verbindung der Formel (I), der Verbindung(en), jeweils enthaltend eine, zwei, drei, vier, fünf, sechs, sieben, acht oder mehr Struktureinheiten (IIa), und der Verbindung(en), jeweils enthaltend eine, zwei, drei, vier, fünf, sechs, sieben, acht oder mehr Struktureinheiten (IIIa), und
- zusätzlich eine, zwei, drei, vier oder mehr Verbindungen umfasst, die unabhängig voneinander jeweils zwei, drei, vier, fünf, sechs, sieben, acht oder mehr Struktureinheiten (IIIa) enthalten, wobei die Gesamtmenge der Verbindung enthaltend jeweils zwei, drei, vier, fünf, sechs, sieben, acht oder mehr Struktureinheiten (IIIa) im Bereich von 20 bis 44 Gew.-% liegt, vorzugsweise in einer Menge von 27 bis 35 Gew.-%, bevorzugt in einer Menge von 28 bis 31 Gew.-%, bezogen auf die Gesamtmasse der Verbindung der Formel (I), der Verbindung(en), jeweils enthaltend eine, zwei, drei, vier, fünf, sechs, sieben, acht oder mehr Struktureinheiten (IIa), und der Verbindung(en), jeweils enthaltend eine, zwei, drei, vier, fünf, sechs, sieben, acht oder mehr Struktureinheiten (IIIa).

Bei der Gesamtmasse der Verbindung der Formel (I), der Verbindung(en), jeweils enthaltend eine, zwei, drei, vier, fünf, sechs, sieben, acht oder mehr Struktureinheiten (IIa), und der Verbindung(en), jeweils enthaltend eine, zwei, drei, vier, fünf, sechs, sieben, acht oder mehr Struktureinheiten (IIIa), handelt es sich um die (Gesamt-)Summe der Massen dieser Verbindungen.

Die Erfindung betrifft zudem die Verwendung eines erfindungsgemäßen Mehrkomponentenklebstoffes zum nicht-therapeutischen Zusammenkleben von zwei Oberflächen.

Die Erfindung betrifft auch ein Verfahren zur Herstellung eines Adhäsivhydrogels umfassend die folgenden Schritte:
- Bereitstellen oder Herstellen eines erfindungsgemäßen Mehrkomponentenklebstoffes (wie oben beschrieben; vorzugsweise wie vorstehend als bevorzugt bezeichnet; hinsichtlich bevorzugter Bestandteile ist das weiter oben Gesagte entsprechend),
- Umsetzen lassen der Komponenten des Mehrkomponentenklebstoffes, so dass das Adhäsivhydrogel gebildet wird.

Bevorzugt gilt für das oben beschriebene erfindungsgemäße Verfahren, dass die Adhäsivhydrogelbildung durch Vernetzung der oben genannten Gruppen X, Y, Z und Z' der Bestandteile des Mehrkomponentenklebstoffes erfolgt.

Besonders bevorzugt gilt für die oben beschriebenen erfindungsgemäßen Verfahren, dass das gebildete Adhäsivhydrogel anschließend mit einem Oxidationsmittel, vorzugsweise mit NaIO₄, H₂O₂ oder mit einer Oxidoreductase, vorzugsweise einer mit der EC-Nummer 1.10 (insbesondere Laccase, Katecholoxidase, Tyrosinase, Phenoloxidase), bevorzugt mit NaIO₄ oder H₂O₂, behandelt wird, sodass eine zusätzliche Quervernetzung erfolgt.

In einigen Ausgestaltungen ist es besonders bevorzugt, das entsprechende Oxidationsmittel bereits beim Vermischen oder kurz nach dem Vermischen aller Komponenten des erfindungsgemäßen Mehrkomponentenklebstoffes hinzuzugeben.

Das mit einem Oxidationsmittel behandelt Adhäsivhydrogel weist besonders gute Eigenschaften auf. Insbesondere wird eine gute Adhäsion an biologischem Gewebe allogenen, heterogenen oder synthetischen Ursprungs und an medizinischen Implantaten und eine gute Barrierewirkung gegen Bakterien erreicht.

Die Erfindung betrifft zudem ein Kit umfassend einen erfindungsgemäßen Mehrkomponentenklebstoff (wie oben beschrieben; vorzugsweise wie vorstehend als bevorzugt bezeichnet; hinsichtlich bevorzugter Bestandteile ist das weiter oben Gesagte entsprechend) und einen oder mehrere weitere Bestandteile.

Bevorzugt ist ein erfindungsgemäßes Kit, bei dem der eine oder die mehreren weiteren Bestandteile ausgewählt ist bzw. sind aus der Gruppe bestehend aus Spritzen, Kanülen, Implantaten, Stents, Kathetern, Applikator, Mischvorrichtung (z.B. statische oder dynamische Mischer) und Oxidationsmittel zur Quervernetzung eines aus dem Mehrkomponentenklebstoff hergestellten Adhäsivhydrogels.

Die Erfindung betrifft auch ein Verfahren zum therapeutischen oder nicht-therapeutischen Zusammenkleben von zwei oder mehr Oberflächen, umfassend die folgenden Schritte:
- Bereitstellen oder Herstellen eines erfindungsgemäßen Mehrkomponentenklebstoffes (wie oben beschrieben; vorzugsweise wie vorstehend als bevorzugt bezeichnet; hinsichtlich bevorzugter Bestandteile ist das weiter oben Gesagte entsprechend),
- Bereitstellen oder Herstellen von zwei oder mehr Oberflächen,
- Aufbringen einer Mischung der Komponenten des hergestellten oder bereitgestellten Mehrkomponentenklebstoffes auf eine, zwei oder mehrere der bereitgestellten oder hergestellten Oberflächen,
- Herstellen eines Verbunds umfassend zwei oder mehr der bereitgestellten oder hergestellten Oberflächen und zwischen diesen Oberflächen und diese verbindend eine Mischung der Komponenten des hergestellten oder bereitgestellten Mehrkomponentenklebstoff
- Umsetzen lassen der Mischung der Komponenten des Mehrkomponentenklebstoffes im besagten Verbund, sodass ein Adhäsivhydrogel entsteht und die Oberflächen des Verbundes zusammengeklebt werden.

Bevorzugt ist ein erfindungsgemäßes Verfahren, bei dem zumindest eine der bereitgestellten oder hergestellten Oberflächen die Oberfläche eines Elements mit einem Titangehalt von mehr als 89 Gew.-% ist, bezogen auf die Gesamtmasse des Elements.

Die Erfindung betrifft auch ein Adhäsivhydrogel herstellbar aus einem erfindungsgemäßen Mehrkomponentenklebstoff (wie oben beschrieben; vorzugsweise wie vorstehend als bevorzugt bezeichnet; hinsichtlich bevorzugter Bestandteile ist das weiter oben Gesagte entsprechend), vorzugsweise hergestellt nach einem erfindungsgemäßen Verfahren zur Herstellung eines Adhäsivhydrogels.

Die Erfindung betrifft ebenfalls einen Artikel umfassend zwei zusammengeklebte Oberflächen, zwischen denen ein aus einem erfindungsgemäßen Mehrkomponentenklebstoff (wie oben beschrieben; vorzugsweise wie vorstehend als bevorzugt bezeichnet; hinsichtlich bevorzugter Bestandteile ist das weiter oben Gesagte entsprechend) herstellbares Adhäsivhydrogel angeordnet ist und den Klebeverbund bewirkt.

Die Erfindung betrifft ebenfalls einen Artikel umfassend eine Oberfläche, auf welcher ein aus einem erfindungsgemäßen Mehrkomponentenklebstoff (wie oben beschrieben; vorzugsweise wie vorstehend als bevorzugt bezeichnet; hinsichtlich bevorzugter Bestandteile ist das weiter oben Gesagte entsprechend) herstellbares Adhäsivhydrogel in Klebeverbindung angeordnet ist.

Die Erfindung betrifft ebenfalls eine Verbindung der Formel (IV) (SEQ ID NO: 16)

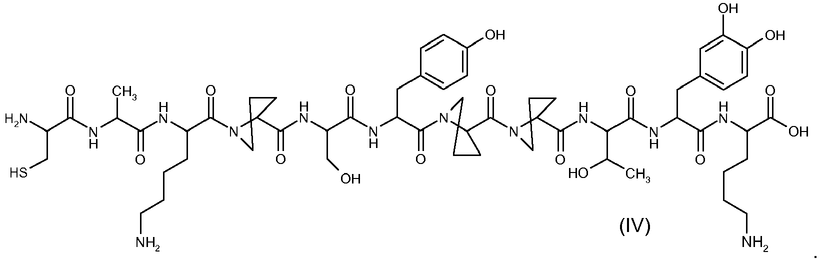

Die nachfolgenden Beispiele erläutern die Erfindung; sofern nicht anders angegeben, beziehen sich alle Angaben auf das Gewicht.

### Beispiele:

### Beispiel 1. Herstellung einer erfindungsgemäßen Verbindung der Formel (IV) (Dekapeptid) (SEQ ID NO: 16):

Für die Synthese wurde ein mit Fmoc-Aminosäuren vorbeladenes TCP-Polystyrolharz an einem 433A Festphasenpeptidsynthesizer (SPPS) der Firma Applied-Biosystems (Farmington, USA) verwendet. Die Synthesen wurden im 0,1 mmol Maßstab durchgeführt. Substitutionsgrad für Lysin = 0,54 mmol/g Es wurde nach dem FastMoc-Protokoll gearbeitet. Dieses Protokoll nutzt die orthogonale Schutzgruppenstrategie: Das -Amin wird mit einer temporären Schutzgruppe ausgestattet. Diese Funktion erfüllt das Fluorenylmethoxycarbonyl (Fmoc). Gleichzeitig sind Aminosäuren, die über reaktive Gruppen an ihren Seitenketten verfügen, mit permanenten Schutzgruppen gegen die Synthesechemie gefeit. Der Fmoc-geschützte N-Terminus der eingesetzten L-Aminosäuren wurde nach jedem Kopplungszyklus und wenn die Harze bereits mit Aminosäure beladen eingesetzt wurden, zusätzlich vor der ersten Kopplung mit 20 % Piperidin in NMP entschützt. Die basenstabilen Seitenkettenschutzgruppen wurden nach der Festphasensynthese entfernt. Die Kopplung einer Aminosäure an die am Harz heranwachsende Sequenz erfolgt durch einen nukleophilen Angriff des N-Terminus am Carbonylkohlenstoff der zu koppelnden Aminosäure. Dieser Kohlenstoff wurde zuvor der standardisierten Aktivierung mit HBTU oder mit HATU in NMP unterzogen. Die Abspaltung des Harzes und der permanenten Schutzgruppen erfolgte in einem Schritt durch die Zugabe von 400 µL eines Trifluoressigsäure/Triisopropylsilan/Wasser-Gemisches im Verhältnis (18:1:1). Das abgespaltene Peptid wurde nach 2 Stunden durch einen Spritzenfillter (0,45 µm) in eiskalten tert-Butyl-Methylether (MTBE) überführt. Das darin präzipitierte Peptid wurde weitere zwei Stunden bei -20°C nachgefällt. Im Anschluss wurde die Lösung zentrifugiert. Der Rückstand wurde in Wasser gelöst, in einen Kolben überführt und lyophilisiert. Man erhält die Verbindung der Formel (IV) (nachfolgend auch Dekapeptid) in hoher Reinheit.

### Beispiel 2: Herstellung eines erfindungsgemäßen Mehrkomponentenklebstoffes und Herstellung eines Adhäsivhvdrogels:

In je einem 500µl-Mikroreaktionsgefäß werden 15 mg (ca. 7,5 µmol) 4-Arm-PEG-SH (Verbindung VI; M_{w} = 2000 g/mol; (4 arm PEG SH (pentaerythritol))) in 150 µl 0,1M Carbonatpuffer (mit 1M HCl auf pH 7,5 eingestellt) und 7 mg (ca. 3,5 µmol) Mal-PEG-Mal (Verbindung VIII; M_{w} = 1930 g/mol; alpha,omega-Bis-maleinimido poly(ethylenglykol)) in 200 µl 0,1M Carbonatpuffer (mit 1M HCl auf pH 7,5 eingestellt) gelöst. Zu der Lösung aus Mal-PEG-Mal wird eine weitere Lösung bestehend aus 1mg (0,7 µmol) Dekapeptid (Verbindung (IV)) gelöst in 50 µl 0,1M Carbonatpuffer (der pH-Wert der fertigen Lösung wird mit ca. 1 µl NaOH-Lösung auf pH 7,5 eingestellt) hinzugegeben und mit einem Vortex-Mixer gut verrührt. Die zwei hergestellten Lösungen bilden einen erfindungsgemäßen Mehrkomponentenklebstoff. Nach 10 Minuten Rührzeit werden die beiden Lösungen bestehend aus a) dem Mal-PEG-Mal sowie dem Dekapeptid und b) 4-Arm-PEG-SH mit Hilfe von zwei Pipetten möglichst gleichzeitig auf ein Substrat appliziert. Es bildet sich ein Adhäsivhydrogel aus, das nach ca. 5 Minuten seine Endfestigkeit erreicht.

### Beispiel 3: Herstellung eines erfindungsgemäßen Mehrkomponentenklebstoffes und Herstellung eines Adhäsivhydrogels mit Quervernetzung:

In je einem 500µl-Mikroreaktionsgefäß werden 15 mg (ca. 7,5 µmol) 4-Arm-PEG-SH (Verbindung VI; M_{w} = 2000 g/mol; (4 arm PEG SH (pentaerythritol))) in 150 µl 0,1M Carbonatpuffer (mit 1M HCl auf pH 7,5 eingestellt) und 7 mg (ca. 3,5 µmol) Mal-PEG-Mal (Verbindung VIII; M_{w} = 1930 g/mol; alpha, omega-Bis-maleinimido poly(ethylenglykol)) in 200 µl 0,1M Carbonatpuffer (mit 1M HCl auf pH 7,5 eingestellt) gelöst. Zu der Lösung aus Mal-PEG-Mal wird eine weitere Lösung bestehend aus 1mg (0,7 µmol) Dekapeptid (Verbindung (IV)) gelöst in 50 µl 0,1M Carbonatpuffer (der pH-Wert der fertigen Lösung wird mit ca. 1 µl NaOH-Lösung auf pH 7,5 eingestellt) hinzugegeben und mit einem Vortex-Mixer gut verrührt. Die zwei hergestellten Lösungen bilden einen erfindungsgemäßen Mehrkomponentenklebstoff. Nach 10 Minuten Rührzeit werden die beiden Lösungen bestehend aus a) dem Mal-PEG-Mal sowie dem Dekapeptid und b) 4-Arm-PEG-SH mit Hilfe von zwei Pipetten möglichst gleichzeitig auf ein Substrat appliziert. Unmittelbar nach der Applikation werden 10µl einer gesättigten Natriumperiodat-Lösung zu dem sich bildenden Adhäsivhydrogel pipettiert. Es bildet sich ein Adhäsivhydrogel aus, das nach ca. 5 Minuten seine Endfestigkeit erreicht.

### Beispiel 4: Haftfestigkeit eines erfindungsgemäßen Adhäsivhvdrogels:

Ein gemäß Beispiel 2 hergestellter Mehrkomponentenklebstoff wird auf ein frisch entnommenes Stück Schweinegingiva als Substrat appliziert und unmittelbar nach der Applikation mit einem Titansubstrat belegt, sodass ein Haftverbund zwischen Titansubstrat und Schweinegingiva gebildet wird. Es bildet sich ein Adhäsivhydrogel aus, das nach ca. 5 Minuten seine Endfestigkeit erreicht.

In einem Zugversuch wird die Zugbelastbarkeit des gebildeten Adhäsivhydrogels zwischen dem Titansubstrat und der Schweinegingiva geprüft.

Das gebildete Adhäsivhydrogel hat sich für die angedachten Zwecke als ausreichend zugbelastbar erwiesen.

### Beispiel 5: Haftfestigkeit eines erfindungsgemäßen Adhäsivhvdroqels:

Ein gemäß Beispiel 2 hergestellter Mehrkomponentenklebstoff wird auf ein frisch entnommenes Stück Schweinegingiva als Substrat appliziert. Unmittelbar nach der Applikation werden 10µl einer gesättigten Natriumperiodat-Lösung zu dem sich bilden Adhäsivhydrogel pipettiert. Unmittelbar nach der Applikation der Natriumperiodat-Lösung wird der Mehrkomponentenklebstoff (bzw. das sich ausbildende Adhäsivhydrogel) mit einem Titansubstrat belegt, sodass ein Haftverbund zwischen Titansubstrat und Schweinegingiva gebildet wird. Es bildet sich ein Adhäsivhydrogel aus, das nach ca. 5 Minuten seine Endfestigkeit erreicht.

In einem Zugversuch wird die Zugbelastbarkeit des gebildeten Adhäsivhydrogels zwischen dem Titansubstrat und der Schweinegingiva geprüft.

Das gebildete Adhäsivhydrogel hat sich für die angedachten Zwecke als ausreichend zugbelastbar erwiesen.

### Vergleichsbeispiel 1: Herstellung eines Hydrogels aus einem Zweikomponentensystem:

In je einem 500µl-Mikroreaktionsgefäß werden 7 mg (ca. 3,5 µmol) Mal-PEG-Mal (Verbindung VIII; M_{w} = 2000 g/mol) und 15 mg (ca. 7,5 µmol) 4-Arm-PEG-SH (Verbindung VI; M_{w} = 2000 g/mol) in je 200 µl 0,1M Carbonatpuffer (mit 1M HCl auf pH 7,5 eingestellt) gelöst. Die beiden Lösungen bestehend aus a) dem Mal-PEG-Mal und b) dem 4-Arm-PEG-SH werden mit Hilfe von zwei Pipetten möglichst gleichzeitig auf ein Substrat appliziert. Nach ca. 5 Minuten hat das gebildete Hydrogel seine Endfestigkeit erreicht.

### Vergleichsbeispiel 2: Haftfestigkeit eines Hydrogels:

Ein Gemäß Vergleichsbeispiel 1 hergestellte Zweikomponentensystem wird auf ein frisch entnommenes Stück Schweinegingiva als Substrat appliziert und unmittelbar nach der Applikation mit einem Titansubstrat belegt, sodass ein Haftverbund zwischen Titansubstrat und Schweinegingiva gebildet wird. Nach ca. 5 Minuten hat das gebildete Hydrogel seine Endfestigkeit erreicht.

In einem Zugversuch wird die Zugbelastbarkeit des gebildeten Hydrogels zwischen dem Titansubstrat und der Schweinegingiva geprüft.

Das gebildete Hydrogel hat für die angedachten Zwecke keine ausreichende Zugbelastbarkeit.

## Patentansprüche

1. Mehrkomponentenklebstoff zur Herstellung eines Adhäsivhydrogels, umfassend
a) eine Verbindung der Formel (I) 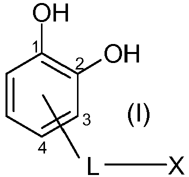 wobei
X ausgewählt ist aus der Gruppe bestehend aus SH, NH₂ und alpha,beta-ungesättigten Carbonylgruppen und
L ein Linker ist, der die 1,2-Dihydroxyphenyl-Einheit mit X verknüpft, wobei der Linker L in 3- oder 4-Position mit der 1,2-Dihydroxyphenyl-Einheit verbunden ist, und die Anzahl der Kettenglieder des Linkers L im Bereich von 5 bis 65 Kettengliedern liegt und der Linker L in der Verbindung der Formel (I) ein, zwei oder mehr Struktureinheiten enthält, ausgewählt aus der Gruppe von Aminosäuresequenzen bestehend aus Alanin-Lysin, Lysin-Prolin, Prolin-Serin, Serin-Tyrosin, Tyrosin-Prolin, Prolin-Prolin, Prolin-Threonin, Alanin-Lysin-Prolin, Lysin-Prolin-Serin, Prolin-Serin-Tyrosin, Serin-Tyrosin-Prolin, Tyrosin-Prolin-Prolin, Prolin-Prolin-Threonin, Alanin-Lysin-Prolin-Serin (SEQ ID NO: 1), Lysin-Prolin-Serin-Tyrosin (SEQ ID NO: 2), Prolin-Serin-Tyrosin-Prolin (SEQ ID NO: 3), Serin-Tyrosin-Prolin-Prolin (SEQ ID NO: 4), Tyrosin-Prolin-Prolin-Threonin (SEQ ID NO: 5), Alanin-Lysin-Prolin-Serin-Tyrosin (SEQ ID NO: 6), Lysin-Prolin-Serin-Tyrosin-Prolin (SEQ ID NO: 7), Prolin-Serin-Tyrosin-Prolin-Prolin (SEQ ID NO: 8), Serin-Tyrosin-Prolin-Prolin-Threonin (SEQ ID NO: 9), Alanin-Lysin-Prolin-Serin-Tyrosin-Prolin (SEQ ID NO: 10), Lysin-Prolin-Serin-Tyrosin-Prolin-Prolin (SEQ ID NO: 11), Prolin-Serin-Tyrosin-Prolin-Prolin-Threonin (SEQ ID NO: 12), Alanin-Lysin-Prolin-Serin-Tyrosin-Prolin-Prolin (SEQ ID NO: 13), Lysin-Prolin-Serin-Tyrosin-Prolin-Prolin-Threonin (SEQ ID NO: 14) und Alanin-Lysin-Prolin-Serin-Tyrosin-Prolin-Prolin-Threonin (SEQ ID NO: 15)
und
b) eine, zwei, drei, vier oder mehr Verbindungen,
jeweils enthaltend eine, zwei, drei, vier, fünf, sechs, sieben, acht oder mehr Struktureinheiten (IIa) 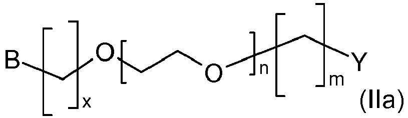 wobei in jeder der Struktureinheiten (IIa) unabhängig von der Bedeutung in anderen Struktureinheiten (IIa)
B ein Verknüpfungspunkt ist,
n eine ganze Zahl größer oder gleich 1 ist, x 0, 1, 2, 3, 4, 5, 6, 7 oder 8 ist und
m 0, 1, 2, 3, 4, 5, 6, 7, oder 8 ist.Y ausgewählt ist aus der Gruppe bestehend aus SH, NH₂ und alpha,beta-ungesättigten Carbonylgruppen und umsetzbar ist mit der Gruppe X unter Ausbildung zumindest einer kovalenten Bindung,
wobei
(i) eine, zwei, drei, vier oder mehr der Verbindungen, die jeweils eine, zwei, drei, vier, fünf, sechs, sieben, acht oder mehrere Struktureinheiten (IIa) enthalten, zusätzlich jeweils einen Rest Z umfassen, der unabhängig von jedem anderen Rest Z einer anderen Verbindung ausgewählt ist aus der Gruppe bestehend aus SH, NH₂ und alpha,beta-ungesättigten Carbonylgruppen und umsetzbar ist mit einer Gruppe Y unter Ausbildung zumindest einer kovalenten Bindung,
wobei in oder als Bestandteil (b) umfasst sind eine, zwei, drei, vier, fünf, sechs, sieben, acht oder mehr Verbindungen ausgewählt aus Verbindungen der Formel (V) 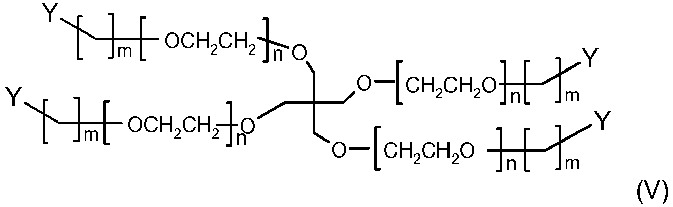 wobei
n in jeder der vier Seitenketten unabhängig von der Bedeutung von n in den jeweils anderen Seitenketten eine ganze Zahl größer oder gleich 1 ist und
m in jeder der vier Seitenketten unabhängig von der Bedeutung von m in den jeweils anderen Seitenketten 0, 1, 2, 3, 4, 5, 6, 7, oder 8 ist
und
Y in jeder der vier Seitenketten unabhängig von der Bedeutung von Y in den jeweils anderen Seitenketten ausgewählt ist aus der Gruppe bestehend aus SH, NH₂ und alpha,beta-ungesättigten Carbonylgruppen und umsetzbar ist mit der Gruppe X unter Ausbildung zumindest einer kovalenten Bindung.
und/oder
(ii) der Mehrkomponentenklebstoff zusätzlich eine, zwei, drei, vier oder mehr Verbindungen umfasst, die unabhängig voneinander jeweils zwei, drei, vier, fünf, sechs, sieben, acht oder mehr Struktureinheiten (IIIa) 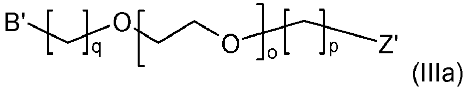 umfassen, wobei in jeder der Struktureinheiten (IIIa) unabhängig von der Bedeutung in anderen Struktureinheiten (IIIa)
B' ein Verknüpfungspunkt ist,
o eine ganze Zahl größer oder gleich 1 ist,
q 0, 1, 2, 3, 4, 5, 6, 7 oder 8 ist
p 0, 1, 2, 3, 4, 5, 6, 7 oder 8 ist, Z' ausgewählt ist aus der Gruppe bestehend aus SH, NH₂ und alpha,beta-ungesättigten Carbonylgruppen und umsetzbar ist mit einer Gruppe Y unter Ausbildung zumindest einer kovalenten Bindung,
umfassend in oder als Verbindung umfassend die Struktureinheit (IIIa) eine, zwei, drei, vier oder mehr Verbindungen ausgewählt aus Verbindungen gemäß Formel (VII) 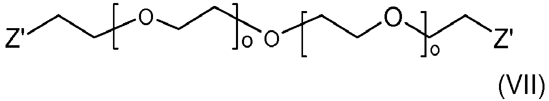 wobei
o in jeder der zwei Seitenketten unabhängig von der Bedeutung von o in den jeweils anderen Seitenketten eine ganze Zahl größer oder gleich 1 ist und
Z' jeder der zwei Seitenketten unabhängig von der Bedeutung von Z' in den jeweils anderen Seitenketten ausgewählt ist aus der Gruppe bestehend aus SH, NH₂ und alpha,beta-ungesättigten Carbonylgruppen und umsetzbar ist mit einer Gruppe Y unter Ausbildung zumindest einer kovalenten Bindung.

2. Mehrkomponentenklebstoff nach Anspruch 1, zusätzlich umfassend
c) einen Puffer bestehend aus einer Puffersäure und einer korrespondierenden Pufferbase, wobei der pK_{B} der Pufferbase nicht kleiner ist als 2, vorzugsweise nicht kleiner ist als 4
und/oder
d) eine oder mehrere Basen mit einem pK_{B} < 1.

3. Mehrkomponentenklebstoff nach einem der Ansprüche 1 oder 2, wobei die Verbindung der Formel (I) die Struktur gemäß der Formel (IV) besitzt

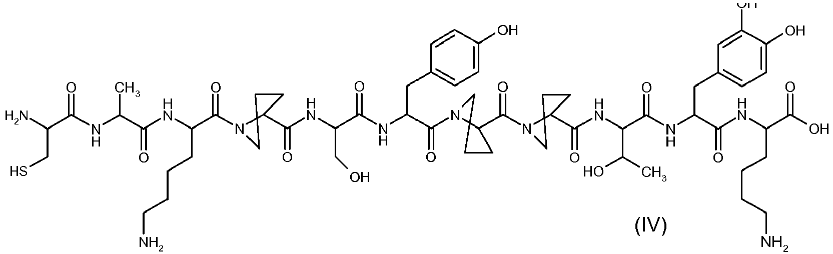

4. Mehrkomponentenklebstoff nach einem der Ansprüche 1 bis 3, zur Anwendung in einem Verfahren zur chirurgischen oder therapeutischen Behandlung des menschlichen oder tierischen Körpers.

5. Verwendung eines Mehrkomponentenklebstoffes nach einem der Ansprüche 1 bis 4 zum nicht-therapeutischen Zusammenkleben von zwei Oberflächen.

6. Verfahren zur Herstellung eines Adhäsivhydrogels umfassend die folgenden Schritte:
- Bereitstellen oder Herstellen eines Mehrkomponentenklebstoffes nach einem der Ansprüche 1 bis 4,
- Umsetzen lassen der Komponenten des Mehrkomponentenklebstoffes, so dass das Adhäsivhydrogel entsteht.

7. Kit umfassend einen Mehrkomponentenklebstoff nach einem der Ansprüche 1 bis 4 und einen oder mehrere weiteren Bestandteile.

8. Kit nach Anspruch 7, wobei der eine oder die mehreren weiteren Bestandteile ausgewählt ist bzw. sind aus der Gruppe bestehend aus Spritzen, Kanülen, Implantaten, Stents, Kathetern und Oxidationsmittel zur Quervernetzung eines aus dem Mehrkomponentenklebstoff hergestellten Adhäsivhydrogels.

9. Adhäsivhydrogel herstellbar aus einem Mehrkomponentenklebstoff nach einem der Ansprüche 1 bis 4, vorzugsweise hergestellt nach einem Verfahren nach Anspruch 6.

## Claims

1. A multi-component adhesive for producing an adhesive hydrogel, comprising
a) a compound of Formula (I) 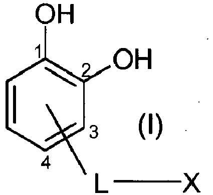 wherein
X is selected from the group consisting of SH, NH₂ and alpha,beta-unsaturated carbonyl groups, and
L is a linker which links the 1,2-dihydroxyphenyl unit with X, wherein the linker L is connected to the 1,2-dihydroxyphenyl unit in the 3- or 4-position, and the number of chain members of the linker L lies in the range from 5 to 65 chain members, and the linker L in the compound of Formula (I) contains one, two or more structural units, selected from the group of amino acid sequences consisting of alanine-lysine, lysine-proline, proline-serine, serine-tyrosine, tyrosine-proline, proline-proline, proline-threonine, alanine-lysine-proline, lysine-proline-serine, proline-serine-tyrosine, serine-tyrosine-proline, tyrosine-proline-proline, proline-proline-threonine, alanine-lysine-proline-serine (SEQ ID NO: 1), lysine-proline-serine-tyrosine (SEQ ID NO: 2), proline-serine-tyrosine-proline (SEQ ID NO: 3), serine-tyrosine-proline-proline (SEQ ID NO: 4), tyrosine-proline-proline-threonine (SEQ ID NO: 5), alanine-lysine-proline-serine-tyrosine (SEQ ID NO: 6), lysine-proline-serine-tyrosine-proline (SEQ ID NO: 7), proline-serine-tyrosine-proline-proline (SEQ ID NO: 8), serine-tyrosine-proline-proline-threonine (SEQ ID NO: 9), alanine-lysine-proline-serine-tyrosine-proline (SEQ ID NO: 10), lysine-proline-serine-tyrosine-proline-proline (SEQ ID NO: 11), proline-serine-tyrosine-proline-proline-threonine (SEQ ID NO: 12), alanine-lysine-proline-serine-tyrosine-proline-proline (SEQ ID NO: 13), lysine-proline-serine-tyrosine-proline-proline-threonine (SEQ ID NO: 14) and alanine-lysine-proline-serine-tyrosine-proline-proline-threonine (SEQ ID NO: 15)
and
b) one, two, three, four or more compounds,
in each case containing one, two, three, four, five, six, seven, eight or more structural units (IIa) 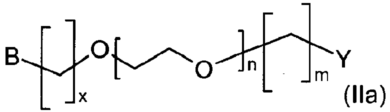 wherein in each of the structural units (IIa), independently of their meaning in other structural units (IIa),
B is a linkage point,
n is a whole number greater than or equal to 1, x is 0, 1, 2, 3, 4, 5, 6, 7 or 8 and
m is 0, 1, 2, 3, 4, 5, 6, 7 or 8, Y is selected from the group consisting of SH, NH₂ and alpha,beta-unsaturated carbonyl groups and can be reacted with the group X, forming at least one covalent bond,
wherein
(i) one, two, three, four or more of the compounds, which in each case contain one, two, three, four, five, six, seven, eight or more structural units (IIa), additionally comprise in each case a radical Z which, independently of every other radical Z of another compound, is selected from the group consisting of SH, NH₂ and alpha,beta-unsaturated carbonyl groups and can be reacted with a group Y, forming at least one covalent bond,
wherein there are comprised in or as constituent (b) one, two, three, four, five, six, seven, eight or more compounds selected from compounds of the formula (V) 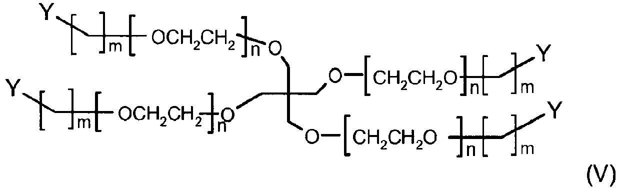 wherein
n in each of the four side chains, independently of the meaning of n in the other side chains in each case, is a whole number greater than or equal to 1, and
m in each of the four side chains, independently of the meaning of m in the other side chains in each case, is 0, 1, 2, 3, 4, 5, 6, 7 or 8
and
Y in each of the four side chains, independently of the meaning of Y in the other side chains in each case, is selected from the group consisting of SH, NH₂ and alpha,beta-unsaturated carbonyl groups and can be reacted with the group X, forming at least one covalent bond.
and/or
(ii) the multi-component adhesive additionally comprises one, two, three, four or more compounds which independently of each other comprise in each case two, three, four, five, six, seven, eight or more structural units (IIIa) 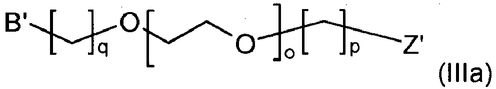 wherein in each of the structural units (IIIa) independently of their meaning in other structural units (IIIa)
B' is a linkage point,
o is a whole number greater than or equal to 1,
q is 0, 1, 2, 3, 4, 5, 6, 7 or 8
p is 0, 1, 2, 3, 4, 5, 6, 7 or 8, Z' is selected from the group consisting of SH, NH₂ and alpha,beta-unsaturated carbonyl groups and can be reacted with a group Y, forming at least one covalent bond,
comprising in or as compound comprising the structural unit (IIIa) one, two, three, four or more compounds selected from compounds according to formula (VII) 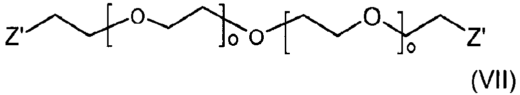 wherein
o in each of the two side chains, independently of the meaning of o in the other side chains in each case, is a whole number greater than or equal to 1, and
Z' [in] each of the two side chains, independently of the meaning of Z' in the other side chains in each case, is selected from the group consisting of SH, NH₂ and alpha,beta-unsaturated carbonyl groups and can be reacted with a group Y, forming at least one covalent bond.

2. A multi-component adhesive according to Claim 1, additionally comprising
c) a buffer consisting of a buffer acid and a corresponding buffer base, wherein the pK_{B} of the buffer base is not less than 2, preferably not less than 4, and/or
d) one or more bases with a pK_{B} of < 1.

3. A multi-component adhesive according to one of Claims 1 or 2, wherein the compound of Formula (I) has the structure in accordance with Formula (IV)

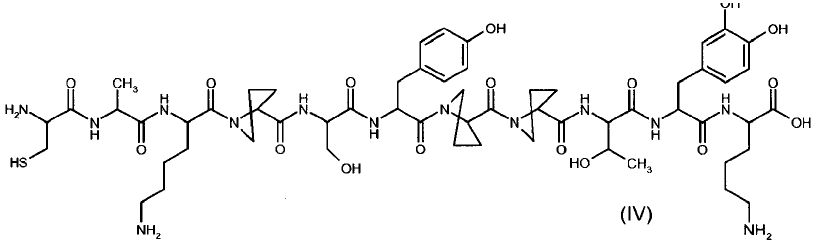

4. A multi-component adhesive according to one of Claims 1 to 3, for application in a method for surgical or therapeutic treatment of the body of humans or animals.

5. Use of a multi-component adhesive according to one of Claims 1 to 4 for the non-therapeutic gluing of two surfaces together.

6. A method for producing an adhesive hydrogel, comprising the following steps:
- providing or producing a multi-component adhesive according to one of Claims 1 to 4,
- allowing the components of the multi-component adhesive to react, so that the adhesive hydrogel is produced.

7. A kit comprising a multi-component adhesive according to one of Claims 1 to 4 and one or more further components.

8. A kit according to Claim 7, wherein the one or more further components(s) is or are selected from the group consisting of syringes, cannulas, implants, stents, catheters and oxidising agents for cross-linking an adhesive hydrogel produced from the multi-component adhesive.

9. An adhesive hydrogel which can be produced from a multi-component adhesive according to one of Claims 1 to 4, preferably produced according to a method according to Claim 6.

## Revendications

1. Adhésif à composants multiples servant à fabriquer un hydrogel adhésif, comprenant
a) un composé de la formule (I) 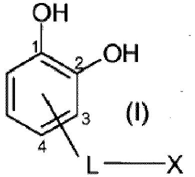 dans lequel
X est choisi parmi le groupe constitué de SH, NH₂ et de groupes carbonyle à insaturation alpha, bêta et
L est un chaînon de liaison, qui relie le motif 1,2-dihydroxyphényle à X, dans lequel le chaînon de liaison L est relié en position 3 ou 4 au motif 1,2-dihydroxyphényle et le nombre des maillons du chaînon de liaison L se situe dans la plage allant de 5 à 65 maillons et le chaînon de liaison L contient dans le composé de la formule (I) un, deux motifs structurels ou plus, choisis parmi le groupe des séquences d'acides aminés constituées d'alanine-lysine, de lysine-proline, de proline-sérine, de sérine-tyrosine, de tyrosine-proline, de proline-proline, de proline-thréonine, d'alanine-lysine-proline, de lysine-proline-sérine, de proline-sérine-tyrosine, de sérine-tyrosine-proline, de tyrosine-proline-proline, de proline-proline-thréonine, d'alanine-lysine-proline-sérine (SEQ ID NO : 1), de lysine-proline-sérine-tyrosine (SEQ ID NO : 2), de proline-sérine-tyrosine-proline (SEQ ID NO : 3), de sérine-tyrosine-proline-proline (SEQ ID NO : 4), de tyrosine-proline-proline-thréonine (SEQ ID NO : 5), d'alanine-lysine-proline-sérine-tyrosine (SEQ ID NO : 6), de lysine-proline-sérine-tyrosine-proline (SEQ ID NO : 7), de proline-sérine-tyrosine-proline-proline (SEQ ID NO : 8), de sérine-tyrosine-proline-proline-thréonine (SEQ ID NO : 9), d'alanine-lysine-proline-sérine-tyrosine-proline (SEQ ID NO : 10), de lysine-proline-sérine-tyrosine-proline-proline (SEQ ID NO : 11), de proline-sérine-tyrosine-proline-proline-thréonine (SEQ ID NO : 12), d'alanine-lysine-proline-sérine-tyrosine-proline-proline (SEQ ID NO : 13), de lysine-proline-sérine-tyrosine-proline-proline-thréonine (SEQ ID NO : 14) et d'alanine-lysine-proline-sérine-tyrosine-proline-proline-thréonine (SEQ ID NO : 15)
et
b) un, deux, trois, quatre composés ou plus,
contenant respectivement un, deux, trois, quatre, cinq, six, sept, huit motifs structurels (IIa) ou plus 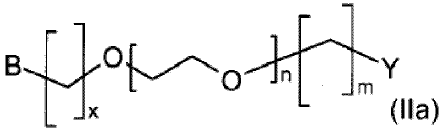 dans lequel dans chacun des motifs structurels (IIa), indépendamment de la signification dans d'autres motifs structurels (IIa)
B est un point de liaison,
n est un nombre entier supérieur ou égal à 1, x est 0, 1, 2, 3, 4, 5, 6, 7 ou 8, et
m est 0, 1, 2, 3, 4, 5, 6, 7 ou 8, Y est choisi parmi le groupe constitué de SH, NH₂ et de groupes carbonyle à insaturation alpha, bêta et peut être mis en réaction avec le groupe X en formant au moins une liaison covalente,
dans lequel
(i) un, deux, trois, quatre composés ou plus, qui contiennent respectivement un, deux, trois, quatre, cinq, six, sept, huit motifs structurels (IIa) ou plus, comprennent en supplément respectivement un radical Z, qui est choisi, indépendamment de chaque autre radical Z d'un autre composé, parmi le groupe constitué de SH, NH₂ et de groupes carbonyle à insaturation alpha, bêta et peut être mis en réaction avec un groupe Y en formant au moins une liaison covalente,
dans lequel sont compris, dans le constituant (b) ou en tant que constituant, un, deux, trois, quatre, cinq, six, sept, huit composés ou plus, choisis parmi des composés de la formule (V) 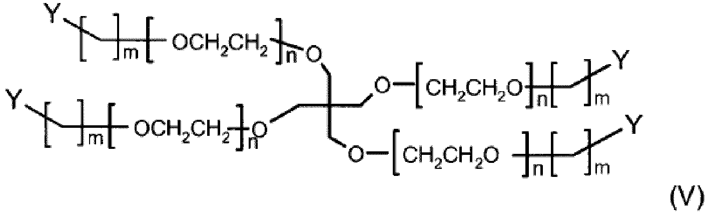 dans lequel
n est, dans chacune des quatre chaînes latérales, indépendamment de la signification de n dans les autres chaînes latérales respectivement, un nombre entier supérieur ou égal à 1, et
m est, dans chacune des quatre chaînes latérales, indépendamment de la signification de m dans les autres chaînes latérales respectivement, 0, 1, 2, 3, 4, 5, 6, 7 ou 8
et
Y est, dans chacune des quatre chaînes latérales, indépendamment de la signification de Y dans les autres chaînes latérales respectivement, choisi parmi le groupe constitué de SH, NH₂ et de groupes carbonyle à insaturation alpha, bêta et peut être mis en réaction avec le groupe X en formant au moins une liaison covalente,
et/ou
(ii) l'adhésif à composants multiples comprend en supplément un, deux, trois, quatre composés ou plus, qui comprennent, indépendamment les uns des autres, respectivement deux, trois, quatre, cinq, six, sept, huit motifs structurels (IIIa) ou plus 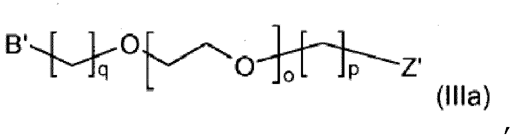 dans lequel dans chacun des motifs structurels (IIIa), indépendamment de la signification dans d'autres motifs structurels (IIIa),
B' est un point de liaison,
o est un nombre entier supérieur ou égal à 1,
q est 0, 1, 2, 3, 4, 5, 6, 7 ou 8,
p est 0, 1, 2, 3, 4, 5, 6, 7, ou 8, Z' est choisi parmi le groupe constitué de SH, NH₂ et de groupes carbonyle à insaturation alpha, bêta et peut être mis en réaction avec un groupe Y en formant au moins une liaison covalente,
comprenant dans ou en tant que composé comprenant le motif structurel (IIIa) un, deux, trois ou quatre composés ou plus choisis parmi des composés selon la formule (VII) 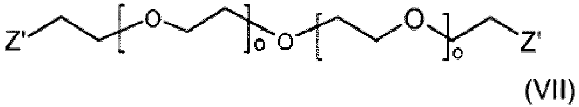 dans lequel
o est, dans chacune des deux chaînes latérales, indépendamment de la signification de o dans les autres chaînes latérales respectivement, un nombre entier supérieur ou égal à 1, et
Z' de chacune des deux chaînes latérales est, indépendamment de la signification de Z' dans les autres chaînes latérales respectivement, choisi parmi le groupe constitué de SH, NH₂ et de groupes carbonyle à insaturation alpha, bêta et peut être mis en réaction avec un groupe Y en formant au moins une liaison covalente.

2. Adhésif à composants multiples selon la revendication 1, comprenant en supplément
c) un tampon constitué d'un acide tampon et d'une base tampon correspondante, dans lequel le pK_{B} de la base tampon n'est pas inférieur à 2, de préférence n'est pas inférieur à 4,
et/ou
d) une ou plusieurs bases avec un pK_{B} < 1.

3. Adhésif à composants multiples selon l'une quelconque des revendications 1 ou 2, dans lequel le composé de la formule (I) possède la structure selon la formule (IV)

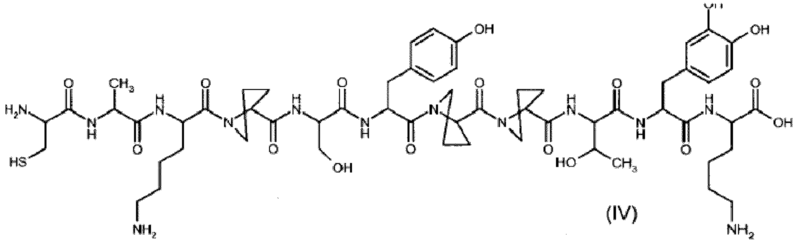

4. Adhésif à composants multiples selon l'une quelconque des revendications 1 à 3, destiné à être mis en oeuvre dans un procédé servant au traitement chirurgical ou thérapeutique du corps humain ou animal.

5. Utilisation d'un adhésif à composants multiples selon l'une quelconque des revendications 1 à 4 servant à regrouper par collage de manière non thérapeutique deux surfaces.

6. Procédé servant à fabriquer un hydrogel adhésif comprenant les étapes suivantes :
- la mise à disposition ou la fabrication d'un adhésif à composants multiples selon l'une quelconque des revendications 1 à 4,
- la réaction des composants de l'adhésif à composants multiples de manière à faire apparaître l'hydrogel adhésif.

7. Ensemble comprenant un adhésif à composants multiples selon l'une quelconque des revendications 1 à 4 et un ou plusieurs autres constituants.

8. Ensemble selon la revendication 7, dans lequel un ou les nombreux autres constituants sont choisis parmi le groupe constitué de seringues, canules, implants, endoprothèses, cathéters et agents d'oxydation servant à la réticulation transversale d'un hydrogel adhésif fabriqué à partir de l'adhésif à composants multiples.

9. Hydrogel adhésif pouvant être fabriqué à partir d'un adhésif à composants multiples selon l'une quelconque des revendications 1 à 4, de préférence fabriqué selon un procédé selon la revendication 6.
